# EUROPEAN PATENT SPECIFICATION

(11) **EP 3 140 420 B1**
(45) Date of publication and mention of the grant of the patent: **11.09.2019**
(21) Application number: 15723184.6
(22) Date of filing: 06.05.2015
(51) Int. Cl.: C12Q 1/6886

(54) **BREAST CANCER EPIGENETIC MARKERS USEFUL IN ANTHRACYCLINE TREATMENT PROGNOSIS**
EPIGENETISCHER BRUSTKREBSMARKER ZUR PROGNOSE DER ANTHRACYCLINBEHANDLUNG
MARQUEURS ÉPIGÉNÉTIQUES DU CANCER DU SEIN UTILES DANS LE PRONOSTIC DE TRAITEMENT À L'ANTHRACYCLINE

(30) Priority: 07.05.2014 EP 14167401
(43) Date of publication of application: 15.03.2017
(73) Proprietor: Université Libre de Bruxelles, 1050 Bruxelles (BE)
(72) Inventor: FUKS, François, B-1160 Bruxelles (BE); JESCHKE, Jana, B-9000 Gent (BE); DEFRANCE, Matthieu, B-1050 Bruxelles (BE); DESMEDT, Christine, B-1860 Meise (BE); BIZET, Martin, B-7134 Ressaix (BE); SOTIRIOU, Christos, B-1180 Bruxelles (BE)
(74) Representative: Vanhalst, Koen
(86) International application number: PCT/EP2015/059954
(87) International publication number: WO 2015/169857

(56) References cited:
- WO-A2-2008/102002
- DE-B3-102010 020 870
- US-A1- 2009 111 707
- M. J. FACKLER ET AL: "Genome-wide Methylation Analysis Identifies Genes Specific to Breast Cancer Hormone Receptor Status and Risk of Recurrence", CANCER RESEARCH, vol. 71, no. 19, 8 August 2011 (2011-08-08), pages 6195-6207, XP055078328, ISSN: 0008-5472, DOI: 10.1158/0008-5472.CAN-11-1630
- GEORG GÃBEL ET AL: "Prognostic significance of methylatedandgenes in blood- and bone marrow plasma of breast cancer patients", BREAST CANCER RESEARCH AND TREATMENT, KLUWER ACADEMIC PUBLISHERS, BO, vol. 130, no. 1, 8 January 2011 (2011-01-08), pages 109-117, XP019958058, ISSN: 1573-7217, DOI: 10.1007/S10549-010-1335-8
- EMELY CASTRO-RIVERA ET AL: "Semaphorin 3B (SEMA3B) Induces Apoptosis in Lung and Breast Cancer, Whereas VEGF 165 Antagonizes This Effect", PROCEEDINGS OF THE NATIONAL ACADEMY OF SCIENCES, vol. 101, no. 31, 3 August 2004 (2004-08-03), pages 11432-11437, XP055146655, ISSN: 0027-8424

## Description

### FIELD OF THE INVENTION

This invention applies to the area of cancer diagnostics and therapeutics. In particular, it relates to hypermethylation of marker genes and their predictive value in the chemotherapy of breast cancer.

### BACKGROUND OF THE INVENTION

With 1 million new cases in the world each year, breast cancer is the commonest malignancy in women and comprises 18% of all female cancers.

Adjuvant chemotherapy and hormonal treatment have substantially reduced the risks of relapse and death in woman with breast cancer. The anthracycline antiobiotics, introduced in the 1980s, are among the most potent cytotoxic agents for breast cancer treatment and thus are components of many (neo)-adjuvant and palliative regimens. In lymph node-positive (LNP) breast cancer, anthracycline-based adjuvant chemotherapy has become the standard of care since the 1990s [1]; 69% of LNP breast cancer patients remained disease-free after five years after treatment with anthracycline-based chemotherapy [2]. Anthracycline-based chemotherapy combinations, such as the cyclophosphamide-epirubicin-fluorouracil (CEF) regimen [3] when compared with the regimen of cyclophosphamide-methotrexate-fluorouracil (CMF), produced on average a further relative reduction in deaths from breast cancer of about 16 percent, which corresponds to a gain in the absolute survival rate at 10 years of approximately 4 percent [4].

Yet, treatment with anthracyclines is linked with both acute and long-term side effects, most notably cardiotoxicity [5] and secondary leukemia [6]. Moreover, retrospective analyses point to clinically significant variations in the magnitude of benefit from adjuvant chemotherapy in different groups of patients [7].

One strategy in the optimization of breast cancer chemotherapy is the search for molecular markers that can help to stratify patients and individualize treatment options. Unfortunately, reliable biomarkers and panels thereof for benefit from anthracycline therapy are still lacking.

The establishment of predictors of the anthracycline treatment outcome would allow the identification and exclusion of individuals who would not benefit from said treatment, and thus to increase the safety of anthracycline treatment. Furthermore, those patients for whom said predicted outcome would be suboptimal, could be recommended for further chemotherapeutic or other treatments. Additionally, biomarkers that can identify LNP patients with a low risk of recurrence after adjuvant anthracycline-based chemotherapy could be useful in avoiding further treatment of this LNP patient group with other chemotherapy agents. These biomarkers could also help personalize decisions regarding whether to incorporate additional chemotherapy agents into adjuvant therapy regimens for individual patients.

Hypermethylation of promoter CpG islands, known as epigenetic alterations, are frequently observed in breast cancer [8-10]. One of the most important features is that DNA methylation appears to be an early event in the etiology of carcinogenesis and to precede apparent malignancy in many cases [11]. The methylation profile is in many cases tissue- and tumor-type specific and is also preserved in purified isolated DNA. Therefore, DNA methylation markers are suitable genetic markers for diagnostic and prognostic purposes of breast cancer.

A goal of the present invention is the finding of further methylation markers that can predict the likelihood of success of anthracycline treatment in breast cancer patients. Another aim of the present invention is the finding of panels of methylation markers with an improved predictive value regarding anthracycline treatment response when compared to the individually known biomarkers or panels of biomarkers.

WO2010/118782 (Université Libre de Bruxelles) discloses methods and tools to predict the efficiency of an anthracycline-based regimen in human patients affected by breast cancer, by detecting the expression of particular genes.

The expression of the T-cell-related marker CD3D was shown to significantly correlate with pathologic complete response (pCR) in breast cancer patients receiving neoadjuvant anthracycline/taxane-based chemotherapy (Denkert et al. 2010. Tumor-associated lymphocytes as an independent predictor of response to neo-adjuvant chemotherapy in breast cancer. J Clin Oncol. 1, 105-113.).

The anthracycline-based score (A-score) combines the topoisomerase II-□ (TOP2A) gene signature, with stroma, and immune signatures related to tumor invasion and immune response. It was found a potentially useful clinical tool to predict outcome of anthracycline treatment (Desmedt et al. 2011. Multifactorial approach to predicting resistance to anthracyclines. J Clin Oncol. 12:1578-1586).

There is evidence that DNA methylation can occur without an effect on gene expression. Widschwendter et al. [12], evaluating a correlation between the methylation status of a potential prognostic marker for cervical and ovarian cancer and gene expression, found that there was no correlation between methylation status and gene expression (see p 412, col 2, p413, col 1, para 1). Suyama et al. [13] previously found that the methylation status of the promoter region did not correlate well with expression of the gene, indicating that other factors were involved in gene expression (see Abstract, p 1117, col 2, p 1118, col 1).

Dietrich et al. [14] disclose that CDO1 methylation was shown to be a strong predictor for distant metastasis in retrospective cohorts of LNP/ER+ breast cancer patients, who had received adjuvant anthracycline-based chemotherapy.

Yuan et al. [15] disclose that BRCA1 methylation status may be a useful predictor for anthracycline-based neoadjuvant chemotherapy in primary breast cancer patients.

Hartmann et al. [16] identified a panel of DNA methylation markers including PITX2, BMP4, FGF4, and C20orf55 that enabled outcome prediction in lymph node-positive, HER-2-negative breast cancer patients treated with anthracycline-based chemotherapy.

Sadr-Nabavi et al. [17] point to a possible predictive value of EFEMP1 expression regarding anthracycline response.

WO2010/007083 (Epigenomics) provides methods, nucleic acids and kits for determining the prognosis of a subject having cancer. It discloses genomic sequences the methylation patterns of which have utility for the improved detection of said disorder, thereby enabling the improved diagnosis and treatment of patients. The genomic sequences of interest are those of CDO1, APC, BMPR1A, CTAGE5, CXCL 12, NCR1, NFATC2, PAX9, POU4F3, and ZBTB16.

US2009/0111707 (Epigenomics) relates to methods for predicting the outcome of anthracycline treatment of cell proliferative disorder patients. This is achieved by determining the expression level of at least one gene selected from the group consisting of PITX2, TFFI, and PLAU. The invention also relates to sequences, oligonucleotides and antibodies which can be used within the described methods. DE102010020870 describes a list of methylation markers, including RASSF1, to predict response to Doxorubicin in breast or ovarian cancer.

Castro-Rivera et al. [18] discloses the link of SEMA3B to cancer development.

Fackler et al. [19] describe a genome wide methylation analysis of breast cancer.

### SUMMARY OF THE INVENTION

The present invention is based on information gathered from pre-therapeutic biopsies of breast cancer patients of the neoadjuvant Trial of Principle (TOP) study using the Infinium® HumanMethylation450k platform. Patients in this trial were treated with anthracycline (epirubicin) monotherapy.

The present inventors could identify DNA methylation signatures, in particular a signature of 29 Infinium® probes corresponding to 24 individual genes (MeTIL signature), a signature of Infinium® probes corresponding to 5 individual genes (5-gene MeTIL signature), and a signature of 3 Infinium® probes corresponding to 3 individual genes (3-gene MeTIL signature), which perform better than the A-score, CD3D marker, and the immune response-related STAT1 and immune response (IR) signatures for predicting response/resistance to anthracyclines in breast cancer. The inventors also found that the identified DNA methylation signatures correlate with Tumor Infiltrating Lymphocytes (TIL) *in vivo.*

Accordingly, the present invention relates to a method for predicting an anthracycline treatment outcome in a patient with breast cancer; said method comprising assaying a test sample from the patient for hypermethylation of the INA gene, combined with all of the genes selected from the group comprising: PTPRCAP, SEMA3B, KLHL6, and RASSF1 (together forming the 5-gene MeTIL gene signature); wherein hypermethylation in said test sample indicates a favorable prognosis.

The present invention also relates to a method for predicting susceptibility to an anthracycline treatment in a patient with breast cancer; said method comprising assaying a test sample from the patient for hypermethylation of the INA gene, combined all of the genes selected from the group comprising: PTPRCAP, SEMA3B, KLHL6, and RASSF1; wherein hypermethylation in said test sample indicates susceptibility to the anthracycline treatment in said patient.

The present invention also relates to a method for selecting a suitable treatment for breast cancer in a patient, said method comprising assaying a test sample from the patient for hypermethylation of the INA gene, combined with all of the genes selected from the group comprising: PTPRCAP, SEMA3B, KLHL6, and RASSF1; wherein:
- if hypermethylation in said test sample is detected, an anthracycline treatment is indicated as a suitable treatment; or
- if minimal or no hypermethylation in said test sample is detected, an anthracycline treatment is not indicated as a suitable treatment.

The present invention relates as well to a method for the stratification of breast cancer patients, said method comprising assaying a test sample from the patient for hypermethylation of the INA gene, combined with all of the genes selected from the group comprising: PTPRCAP, SEMA3B, KLHL6, and RASSF1; wherein hypermethylation in said test sample indicates that said patient will be responsive to an anthracycline treatment.

The present invention further relates to an anthracycline compound for use in the prevention or treatment of a breast cancer in a patient, characterized in that said patient has hypermethylation in the INA gene, combined with all of the genes selected from the group comprising: PTPRCAP, SEMA3B, KLHL6, and RASSF1. Also described herein is a method of prevention or treatment of a patient suffering from breast cancer comprising administering to said patient an effective amount of an anthracycline compound, characterized in that said patient has hypermethylation in at least the INA gene, optionally combined with one, or more of the genes selected from the group comprising: PTPRCAP, SEMA3B, KLHL6, and RASSF1.

The present invention further relates to the use of the hypermethylation of the INA gene, combined with all of the genes selected from the group comprising: PTPRCAP, SEMA3B, KLHL6, and RASSF1, as a marker or preclinical marker, of anthracycline treatment response in a breast cancer patient.

Also described herein are sets of nucleotides and kits for use in the methods of the present invention, as well as their use for determining an anthracycline treatment outcome in a patient with breast cancer; for predicting susceptibility to an anthracycline treatment in a patient with breast cancer; for the stratification of breast cancer patients; or for selecting a suitable treatment for breast cancer in a patient.

### DESCRIPTION OF THE DRAWINGS

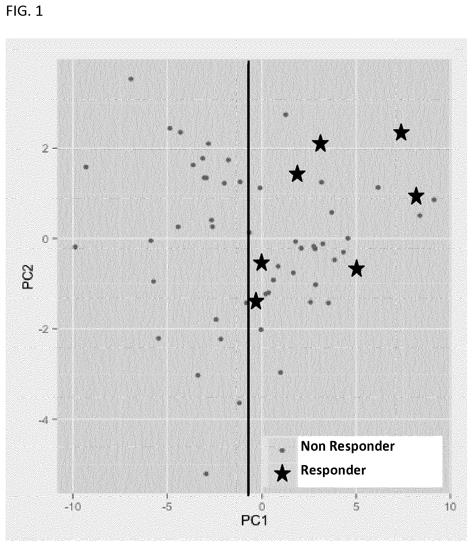

**Figure 1****: PCA score plot of MeTIL signature.** A MeTIL signature of 29 Infinium® probes corresponding to 24 genes was identified upon comparing DNA methylation in T lymphocytes and epithelial breast cancer cells. The signature score was computed using principal component (PC) analysis. Principal component 1 (PC1) is plotted against PC2 and the responders are indicated as black and the non-responders as grey dots. The best threshold for separation of responders from non-responders is a MeTIL Score of -0.4551. This MeTIL score is indicated as vertical line and was obtained from a receiver operating characteristic (ROC) curve.

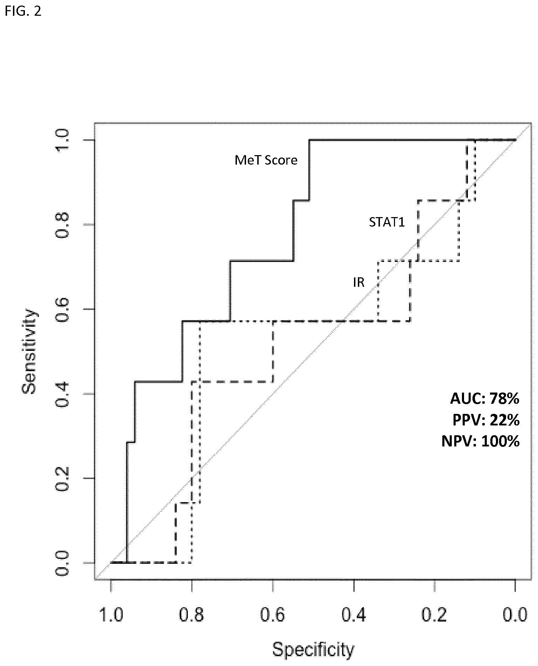

**Figure 2****: ROC curve of MeTIL signature for prediction of pCR.** The receiver operating characteristic (ROC) curve for prediction of pathologic complete response (pCR) in the TOP cohort was computed based on the PCA score of the MeTIL signature (full line). The MeTIL signature predicts for pCR with a specificity of 51% and a sensitivity of 100%. The performance of the MeTIL signature for prediction of pCR is shown in comparison to the performance of the STAT1 (dashed line) and immune response (IR) (dotted line) signatures. Abbreviations: AUC: area under curve. PPV: positive predictive value; NPV: negative predictive value.

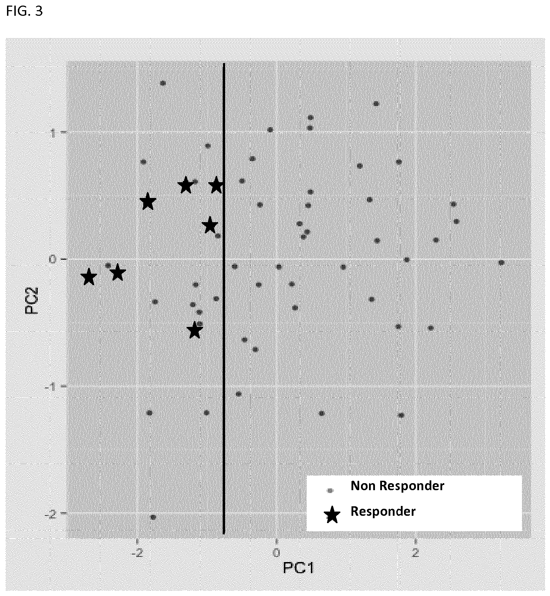

**Figure 3****: PCA score plot of the optimized 3-gene MeTIL signature.** An optimized MeTIL signature of Infinium® probes corresponding to 3 genes (together forming the 3-gene MeTIL gene signature) was identified using a Machine Learning approach, whereby DNA methylation beta value was regressed across response status of the TOP cohort. The optimized 3-gene MeTIL signature score was computed using PC analysis. Principal component 1 (PC1) is plotted against PC2 and the responders are indicated as black and the non-responders as grey dots. The best treshold for separation of responders from non-responders is an optimized 3-gene MeTIL Score of -0.8555. This optimized 3-gene MeTIL score is indicated as vertical line and was obtained from a ROC curve.

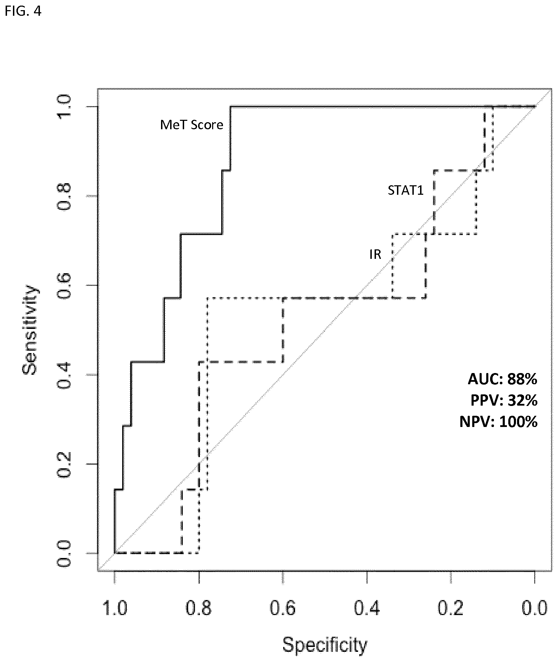

**Figure 4****: ROC curve of the optimized 3-gene MeTIL signature for prediction of pCR.** The ROC curve for prediction of pCR in the TOP cohort was computed based on the PCA score of the optimized MeTIL signature (full line). The optimized MeTIL signature predicts for pCR with a specificity of 73% and a sensitivity of 100%. The performance of the optimized 3-gene MeTIL signature for prediction of pCR is shown in comparison to the performance of STAT1 (dashed line) and immune response (IR) (dotted line) signatures. Abbreviations: AUC: area under curve; PPV: positive predictive value; NPV: negative predictive value.

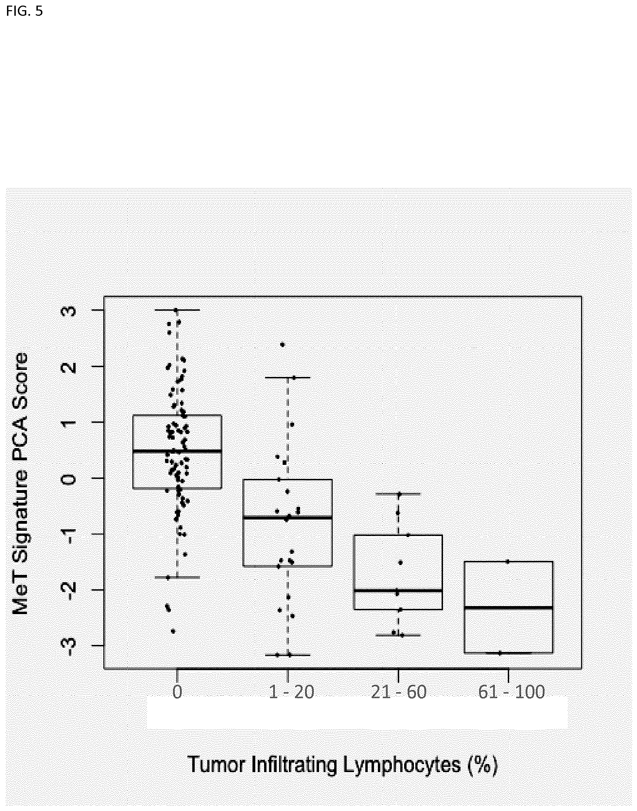

**Figure 5****: The optimized 3-gene MeTIL signature anti-correlates with TIL *in vivo.*** The score of the optimized 3-gene MeTIL signature is plotted against intratumoral lymphocyte infiltration (TIL) (4 categories, in %) in the PNC1 cohort, for which pathological TIL data and Infinum®HumanMethylation450 signatures were available.

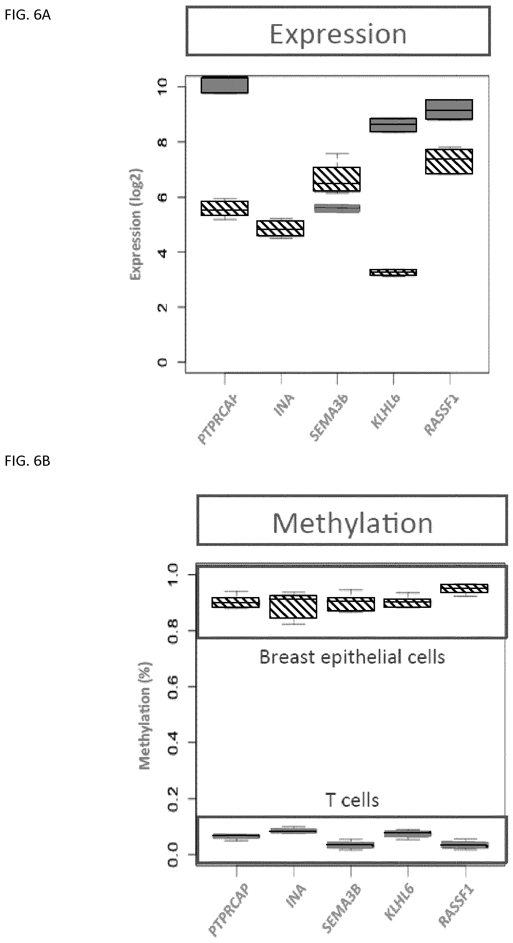

**Figure 6****: Measuring TIL distributions with DNA methylation (5-gene MeTIL signature)** A) Individual markers: INA, PTPRCAP, SEMA3B, KLHL6 and RASSF1 of the 5-gene MeTIL signature show highly differential methylation patterns in epithelial breast cells (barred) versus lymphocytes (grey). B) On the gene expression level inverse patterns appear that are less distinctive between epithelial breast cells (barred) and lymphocytes (grey) than methylation patterns.

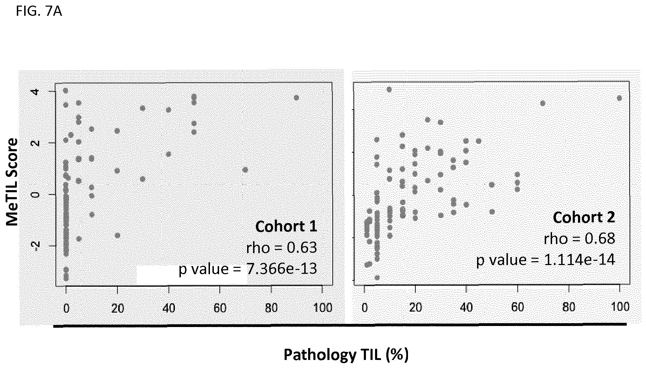

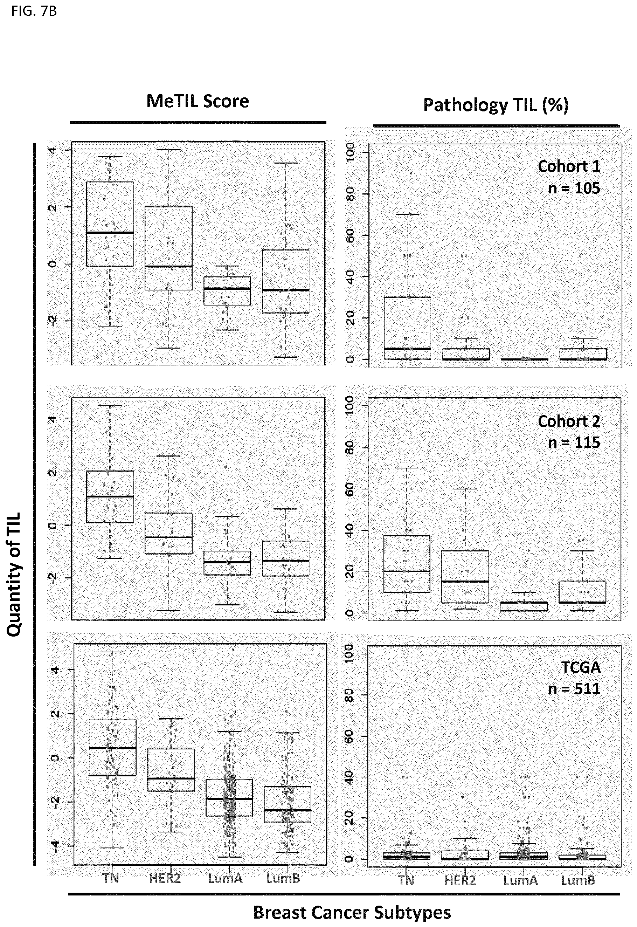

**Figure 7****: Comparison of the 5-gene MeTIL score comprising the combined MeTIL profiles of five genes (INA, PTPRCAP, SEMA3B, KLHL6 and RASSF1) and the known Pathological-TIL (PathoTIL) profile in different breast cancer types, indicating that said 5-gene MeTIL score outperforms the Patho-TIL score in terms of sensitivity.** A) The 5-gene MeTIL signature was transformed into a score and correlated with pathological TIL readings in cohort 1 (discovery cohort; 105 samples) and cohort 2 (independent validation cohort; 115 samples) breast cancer cohorts. The 5-gene MeTIL score values are plotted on the y-axis and pathological TIL readings in % on the x-axis. The Spearman's rank correlation coefficient and its p value are displayed in the lower, right corner of each plot. B) Levels of TIL in breast cancer subtypes based on the 5-gene MeTIL score (left panel) and pathological TIL readings (right panel) in cohort 1 (discovery), cohort 2 and TCGA (both validation) breast cancer cohorts. Note, the 5-gene MeTIL score shows greater differences in TIL levels within (especially in LumA and LumB) and between subtypes than pathological TIL readings suggesting a greater sensitivity of the 5-gene MeTIL signature for detection of TIL.

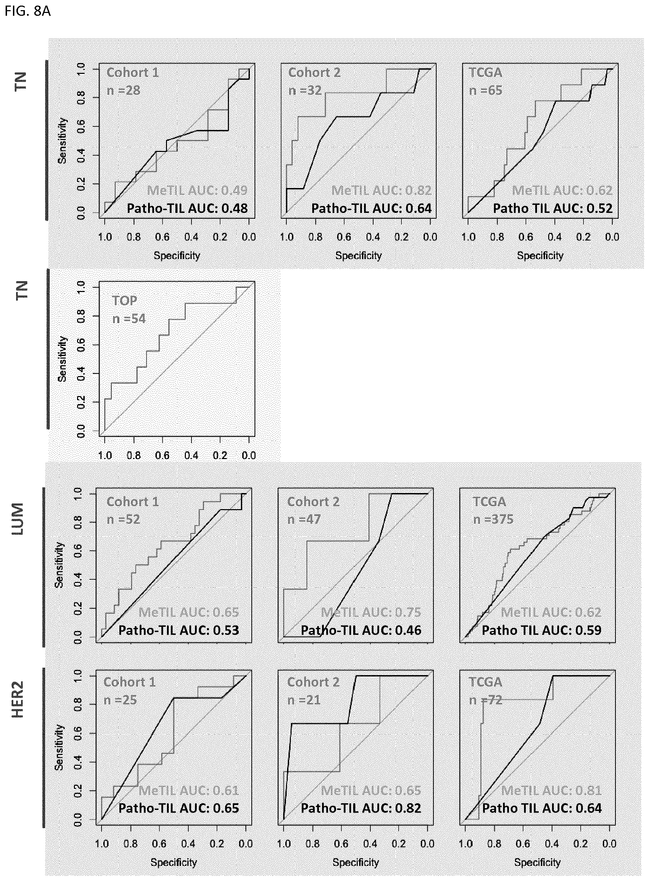

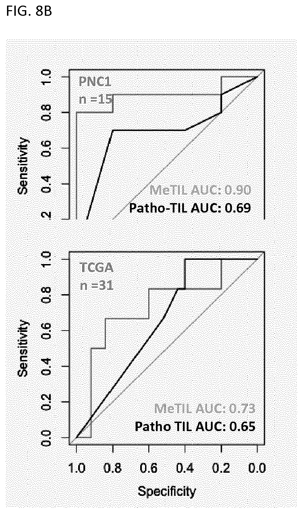

**Figure 8****: The 5-gene MeTIL signature improves prediction of survival and response to anthracycline treatment.** Comparison of the AUC (Area Under the Curve) of the 5-gene MeTIL and PathoTIL in different breast cancer types. **A)** ROC curves for survival are shown for breast cancer subtypes (triple negative (TN), luminal (LUM), HER2-positive (HER2) based on the 5-gene MeTIL score (grey) or pathological TIL readings (black) in various breast cancer cohorts (cohort 1, cohort 2, TCGA, TOP). **B)** ROC curves for survival in the HER2-enriched breast cancer subtype (defined with PAM50 signature) based on the 5-gene MeTIL score (grey) or pathological TIL readings (black) in cohort 1 and TCGA cohort.

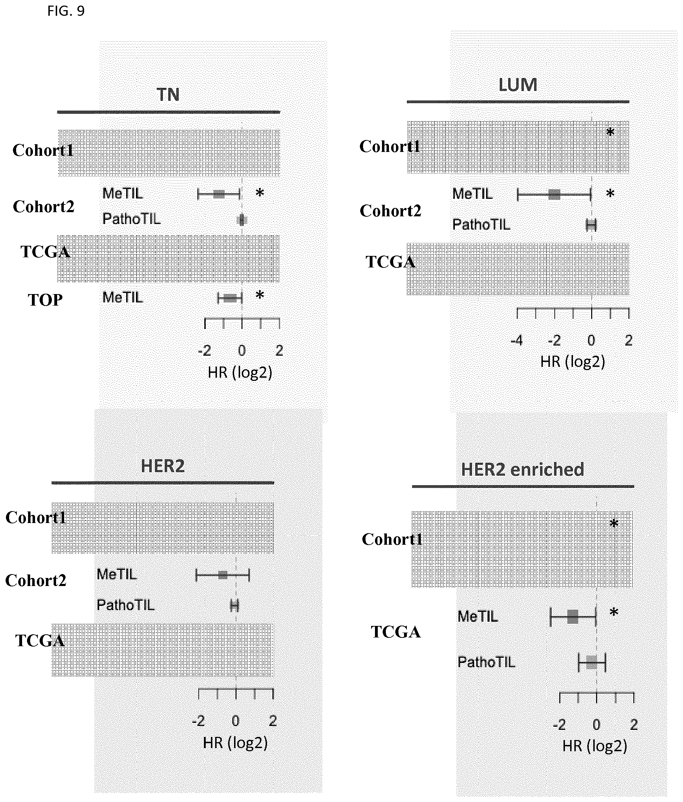

**Figure 9****: The 5-gene MeTIL signature improves prediction of survival and response to anthracycline treatment.** Forest plots by breast cancer subtype showing the log2 value of the hazard ratios (HR) and confidence intervals (CI) for prediction of survival outcome for the 5-gene MeTIL score or pathological TIL readings in various breast cancer cohorts. Asterisks indicates statistical significance (p < 0.05).

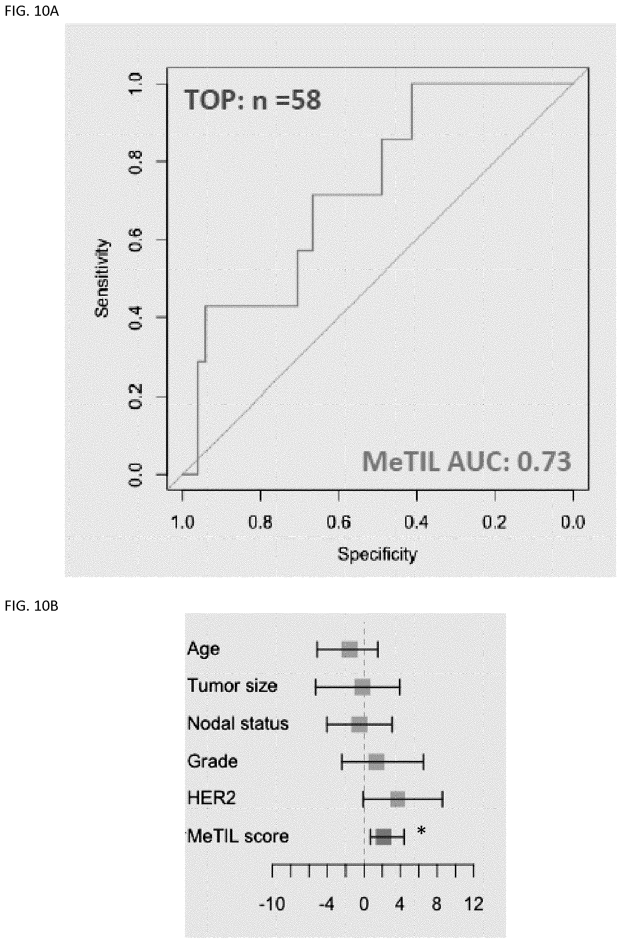

**Figure 10****: The 5-gene MeTIL signature predicts for response to anthracycline treatment. A)** ROC curve for prediction of response to neoadjuvant anthracycline treatment based on the 5-gene MeTIL score in 58 patients of the TOP cohort. **B)** Forest plot showing the log2 value of the odds ratios (OR) and CI of the MeTIL score (bottom row) and various other clinical and pathological relevant variables (others) for prediction of response to neoadjuvant anthracycline treatment in the TOP cohort. Asterisk indicates statistical significance (p < 0.05).

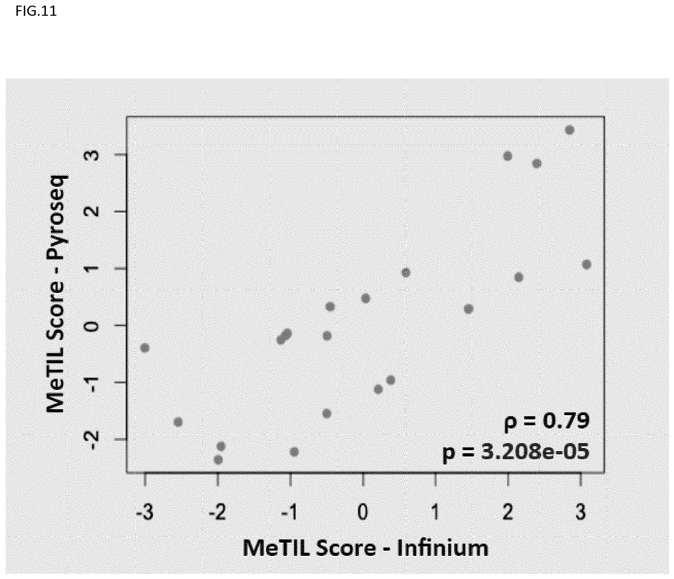

**Figure 11****: Determining the 5-gene MeTIL score in FFPE tumor tissue by bisulfite pyrosequencing.** Scatter plot displaying the correlation between the MeTIL score determined by pyrosequencing in FFPE tumor tissue (y-axis) and the MeTIL score determined by Infinium arrays in fresh-frozen tumor tissue (x-axis). The correlation was established based on 21 patients of cohort 1, for which fresh-frozen and FFPE tissue was available. The Spearman's rank correlation coefficient (rho) and its p value are displayed in the lower, right corner of the plot. The comparison of pyrosequencing on very low amounts of DNA obtained from FFPE tumor tissue to the use of the Infinium methylation microarray technology, shows that pyrosequencing is a cost-efficient alternative for Infinium to analyse the 5-gene MeTIL score in patients.

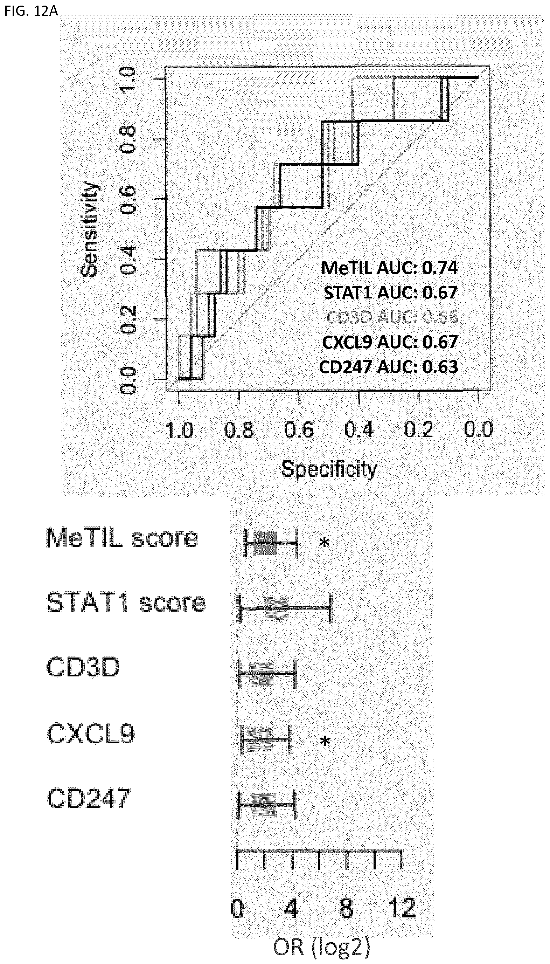

**Figure 12****: The predictive value of the 5-gene MeTIL signature for response to anthracycline treatment in comparison to gene expression-based immune markers.** A) ROC curves for prediction of response to neoadjuvant anthracycline treatment based on the 5-gene MeTIL score or gene expression-based immune markers in 54 patients of the TOP cohort. B) Forest plot showing the log2 value of the odds ratios (OR) and CI of the 5-gene MeTIL score (top row) or gene expression-based immune markers (others) for prediction of response to neoadjuvant anthracycline treatment in the TOP cohort. Asterisks indicates statistical significance (p < 0.05).

### DETAILED DESCRIPTION OF THE INVENTION

As used herein, the singular forms "a", "an", and "the" include both singular and plural referents unless the context clearly dictates otherwise. By way of example, "an antibody" refers to one or more than one antibody; "an antigen" refers to one or more than one antigen.

The terms "comprising", "comprises" and "comprised of" as used herein are synonymous with "including", "includes" or "containing", "contains", and are inclusive or open-ended and do not exclude additional, non-recited members, elements or method steps.

The term "and/or" as used in the present specification and in the claims implies that the phrases before and after this term are to be considered either as alternatives or in combination.

All documents cited in the present specification are hereby incorporated by reference in their entirety. In particular, the teachings of all documents herein specifically referred to are incorporated herein by reference.

The present disclosure relates to a method for predicting an anthracycline treatment outcome in a patient with breast cancer; said method comprising assaying a test sample from the patient for hypermethylation of at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, preferably at least the INA gene, optionally combined with one, or more of the genes selected from the group comprising: PTPRCAP, SEMA3B, KLHL6, and RASSF1 (together the 5-gene signature) wherein hypermethylation in said test sample indicates a favorable prognosis. In another aspect the genes OSM, SEMA3B, and VAV1 are selected (together the 3-gene signature).

The present disclosure relates to a method for predicting susceptibility to an anthracycline treatment in a patient with breast cancer; said method comprising assaying a test sample from the patient for hypermethylation of at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, preferably at least the INA gene, optionally combined with one, or more of the genes selected from the group comprising: PTPRCAP, SEMA3B, KLHL6, and RASSF1 (together the 5-gene signature); wherein hypermethylation in said test sample indicates susceptibility to the anthracycline treatment in said patient. In another aspect, the genes OSM, SEMA3B, and VAV1 are selected (together the 3-gene signature).

The present disclosure also relates to a method for selecting a suitable treatment for breast cancer in a patient, said method comprising assaying a test sample from the patient for hypermethylation of at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, preferably at least the INA gene, optionally combined with one, or more of the genes selected from the group comprising: PTPRCAP, SEMA3B, KLHL6, and RASSF1 (together the 5-gene signature):
- if hypermethylation in said test sample is detected, an anthracycline treatment is indicated as a suitable treatment; or
- if minimal or no hypermethylation in said test sample is detected, an anthracycline treatment is not indicated as a suitable treatment. In another aspect, the genes OSM, SEMA3B, and VAV1 are selected (together the 3-gene signature).

The following Table 1 provides the standard nomenclature, as well as the accession numbers for the genomic and mRNA reference sequences of the marker genes of the present invention, derived all from *Homo sapiens.* Source: National Center for Biotechnology Information (NCBI). The listed accession numbers may be found in the publicly available gene database at http://www.ncbi.nlm.nih.gov.

**Table 1**

| official symbol | official full name | NCBI Reference Sequences | |
|---|---|---|---|
| | | Genomic | mRNA |
| RASSF1 | Ras association (RaIGDS/AF-6) domain family member | GeneID:11186 | NM_007182; NM_170714 |
| SHANK2 | SH3 and multiple ankyrin repeat domains 2 | GeneID:22941 | NM_012309; NM_133266 |
| FAM113B | PC-esterase domain containing 1B | GeneID:91523 | NM_138371; NR_026544 |
| VSX1 | visual system homeobox 1 | GeneID:30813 | NM_014588; NM_199425 |
| DRD4 | dopamine receptor D4 | GeneID:1815 | NM_000797 |
| CD6 | CD6 molecule | GeneID:923 | NM_006725 |
| OSM | oncostatin M | GeneID:5008 | NM_020530 |
| BIN2 | bridging integrator 2 | GeneID:51411 | NM_016293 |
| SEMA3B | sema domain, immunoglobulin domain, secreted, (semaphorin) 3B domain (Ig), short basic | GeneID:7869 | NM_001005914; NM_004636 |
| VAV1 | vav 1 guanine nucleotide exchange factor | GeneID:7409 | NM_005428 |
| MT3 | metallothionein3 | GeneID:4504 | NM_005954 |
| HEM1 | NCK-associated protein 1-like | GeneID:3071 | NM_005337 |
| LCK | lymphocyte-specific protein tyrosine kinase | GeneID:3932 | NM_005356 |
| EDG6 | sphingosine-1-phosphate receptor 4 | GeneID:8698 | NM_003775 |
| KLHL6 | kelch-like family member 6 | GeneID:89857 | NM_130446 |
| ADA | adenosine deaminase | GeneID:100 | NM_000022 |
| PTPRCAP | protein tyrosine phosphatase, receptor type, C-associated protein | GeneID:5790 | NM_005608 |
| CI6orf54 | chromosome 16 open reading frame 54 | GeneID:283897 | NM_175900 |
| INA | internexin neuronal intermediate filament protein, alpha | GeneID:9118 | NM_032727 |
| LAT | linker for activation of T cells | GeneID:27040 | NM_001014989;NM_001014987;NM_014387;NM_00 1014988 |
| SH2D3C | SH2 domain containing 3C | GeneID:10044 | NM_170600 |
| CD3D | CD3d molecule, delta (CD3-TCR complex) | GeneID:915 | NM_001040651;NM_000732 |
| PPP1R16B | protein phosphatase 1, regulatory subunit 16B | GeneID:26051 | NM 015568 - |
| ALDH1A3 | aldehyde dehydrogenase 1 family, member A3 | GeneID:220 | NM 000693 - |

The genomic molecules and transcripts presented herein are not limited to the particular sequences referred to above, but also comprise variants thereof.

The term "anthracycline treatment" relates to the act of "treating", as explained herein below, by administering a composition comprising one or more anthracyclines and/or derivatives thereof. The term "anthracycline treatment" is used interchangeably with synonym phrases like anthracycline-based chemotherapy or "anthracyclines" in its plural form. The term "anthracycline treatment" also relates to anthracycline-based chemotherapy combinations, such as the cyclophosphamide-epirubicin-fluorouracil (CEF) regimen.

The term "anthracycline" refers to the family of anthracycline antibiotic drugs that include but are not limited to mitoxantrone, aclarubicin, daunorubicin, doxorubicin, and epirubicin. Anthracyclines are usually obtained from the fungus *Streptomyces* species, particularly S. *peuticus* var. *caesius,* but synthetic procedures are also encompassed in the present definition. Anthracyclines have a tetrahydronaphthacenedione ring structure attached by a glycosidic linkage to a sugar molecule but variations of the tetrahydronaphthacenedione ring structure and the sugar molecule are also encompassed in the present definition.

Derivatives of anthracyclines are generated by modifications of the backbone including a large number of different side chains, for example having a tetracycline ring structure with an unusual sugar, daunosamine, attached by a glycosidic linkage.

The term "treatment" refers to the act of "treating", a term that is also understood as reverting, relieving or inhibiting the progression of the disease, disorder or condition to which the term is applied to, or of one or several of the symptoms associated to the disease, disorder or condition.

The term "breast cancer" described in the methods or uses of the invention encompasses in principle all cancers of breast-related tissue, including ducts, glands or lobules and infiltrating lymph and/or blood vessels. Specific examples of breast cancer are for example: Ductal Carcinoma In-Situ (DCIS), a type of early breast cancer confined to the inside of the ductal system. Infiltrating Ductal Carcinoma (IDC) is the most common type of breast cancer representing 78% of all malignancies. These lesions appear as stellate (star like) or well-circumscribed (rounded) areas on mammograms. The stellate lesions generally have a poorer prognosis. Medullary Carcinoma accounts for 15% of all breast cancer types. It most frequently occurs in women in their late 40s and 50s, presenting with cells that resemble the medulla (gray matter) of the brain. Infiltrating Lobular Carcinoma (ILC) is a type of breast cancer that usually appears as a subtle thickening in the upper-outer quadrant of the breast. This breast cancer type represents 5% of all diagnosis. Often positive for estrogen and progesterone receptors, these tumors respond well to hormone therapy. Tubular Carcinoma makes up about 2% of all breast cancer diagnosis, tubular carcinoma cells have a distinctive tubular structure when viewed under a microscope. Typically this type of breast cancer is found in women aged 50 and above. It has an excellent 10-year survival rate of 95%. Mucinous Carcinoma (Colloid) represents approximately 1% to 2% of all breast carcinoma. This type of breast cancers main differentiating features are mucus production and cells that are poorly defined. It also has a favorable prognosis in most cases. Inflammatory Breast Cancer (IBC) is a rare and very aggressive type of breast cancer that causes the lymph vessels in the skin of the breast to become blocked. This type of breast cancer is called "inflammatory" because the breast often looks swollen and red, or "inflamed". IBC e.g. accounts for 1% to 5% of all breast cancer cases in the United States. Breast cancer subtypes can furthermore be identified on the basis of gene expression by applying the Subtype Classification Model as described by Desmedt et al., 2008 (Clin. Cancer Res. 14, 5158-5165) and Wirapati et al.,2008 (Breast Cancer Res. 10:R65).

"Breast cancer" as used herein may be of the HER-2-positive type, HER-2-negative type, lymph node-positive (LNP) type, lymph node-negative type, estrogen receptor-positive (ER+) type, ER-negative type, LNP / ER+, LNP / ER+ / HER-2-negative, ER-negative-HER-2-positive type, luminal A-type, luminal B-type, the basal type, or further combinations thereof. The term "lymph node-positive" (LNP), refers to the presence of lymph nodes, which are oval or bean shaped bodies (about 1 - 30 mm in diameter) located along the lymphatic system. The term "HER-2-positive type," "estrogen receptor-positive (ER+) type," when used to describe a cell proliferative disorder means that the proliferating cells express said hormone receptors (Human Epidermal growth factor Receptor 2 and estrogen receptor, respectively). Conversely, the term "HER-2-negative type," "ER-negative type," when used to describe a cell proliferative disorder means that the proliferating cells do not express said hormone receptors. Combinations of hormone receptors expression profiles as well as with other staging classifications are also possible such as: LNP / ER+, LNP / ER+ / HER-2-negative, or the ER-negative-HER-2-positive type, to name a few.

The term "cancer" is meant to encompass both pre-cancer and cancer. Pre-cancer is meant to include those pathologies preceding cancer.

As used herein, a "patient" refers to an animal subject. Among animal subjects are mammals, including primates, such as humans. Preferably, the patient is a human subject. The term "patient" may be used interchangeably with "subject" or "individual".

The term "outcome" refers to the evaluation undertaken to assess the results or consequences of management and procedures used in combatting disease in order to determine the efficacy, effectiveness, safety, practicability, etc., of these interventions in individual cases or series. In the context of the present invention, "outcome" refers to a process of assessing the consequences of treating an individual afflicted with breast cancer with anthracycline-based chemotherapy, i.e. predicting whether said individual is likely to respond or not to said treatment. The methods of the present invention provide a prediction of how a patient's breast cancer will progress when treated with anthracyclines and whether there is chance of recovery.

The methods according to the present invention for predicting an anthracycline treatment outcome comprise predicting one or more (pre)clinical responses such as biological responses (e.g., a cellular response) or clinical responses, particularly pathologic complete response (pCR), increased loco-regional control (LRC), overall survival (OS), disease-free survival (DFS), or disease-specific survival (DSS). The methods of the present invention are particularly suitable for predicting pCR in a patient.
Pathologic complete response (pCR) is defined herein as absence of residual invasive breast carcinoma in the breast and in the axillary nodes.

Locoregional control (LRC) is a primary clinical endpoint measured from the date of treatment randomization to the occurrence of either event or to the date of last follow-up. Locoregional control is defined as local control without any nodal recurrence.

A more commonly used term - overall survival or survival rate - measures death due to any cause (the cancer and any other reason), but is not specific for the disease in question. "Overall survival" or "survival rate" refers to the percentage of subjects in a study who have survived for a defined period of time. This percentage is usually reported together with the period of time since the date of diagnosis or treatment. Typically, overall survival is measured from the day of surgery until tumor-related death.

"Disease-free survival" (DFS) denotes the chances of staying free of disease after a particular treatment for a group of individuals suffering from a cancer. It is the percentage of individuals in the group who are likely to be free of disease after a specified period of time. The time period usually begins at the time of diagnosis, at the start of treatment, or from the day of surgery, and ends at the time of local or distant relapse. Disease-free survival rates are an indication of how effective a particular treatment is. Very often, two treatment strategies are compared on the basis of the disease-free survival that is achieved in similar groups of patients. Disease-free survival is often used with the term overall survival when cancer survival is described.

"Disease-specific survival" (DSS) rate refers to the percentage of people in a study or treatment group who have not died from a specific disease in a defined period of time. The time period usually begins at the time of diagnosis or at the start of treatment and ends at the time of death. Patients who died from causes other than the disease being studied are not counted in this measurement.

The term "susceptibility" as used herein, refers to the quality that predisposes one breast cancer patient to be responsive or reactive to an anthracycline treatment. In the present disclosure, the finding of hypermethylation of at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, preferably at least the INA gene, optionally combined with one, or more of the genes selected from the group comprising: PTPRCAP, SEMA3B, KLHL6, and RASSF1 (together the 5-gene signature) in a test sample from a breast cancer patient indicates that said patient is responsive to the anthracycline treatment. In an alternative aspect,
the genes OSM, SEMA3B, and VAV1 (together the 3-gene signature) can be used. A tumor, including tumor cells, is "susceptible" or "sensitive" (which terms are used interchangeably) to an anthracycline treatment if its rate of growth is inhibited as a result of contact with said anthracycline treatment, compared to its growth in the absence of contact with said anthracycline treatment.

The term "resistance" as used herein, refers to the quality that predisposes one breast cancer patient to a minimal or nul response or reaction to an anthracycline treatment. In the present disclosure, the finding of no or minimal hypermethylation of at least one gene selected from the group comprising: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, preferably at least the INA gene, optionally combined with one, or more of the genes selected from the group comprising: PTPRCAP, SEMA3B, KLHL6, and RASSF1 (together the 5-gene signature) in a test sample from a breast cancer patient indicates that said patient will have no or minimal response to an anthracycline treatment. In an alternative aspect, the genes OSM, SEMA3B, and VAV1 (together the 3-gene signature) can be used. A tumor, including tumor cells, is resistant to an anthracycline treatment if its rate of growth is not inhibited, or inhibited to a very low degree, as a result of contact with said anthracycline treatment when compared to its growth in the absence of contact with said anthracycline treatment.

The present disclosure further relates to a method for the stratification of breast cancer patients, said method comprising assaying a test sample from the patient for hypermethylation of at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, preferably at least the INA gene, optionally combined with one, or more of the genes selected from the group comprising: PTPRCAP, SEMA3B, KLHL6, and RASSF1 (together the 5-gene signature) ; wherein hypermethylation in said test sample indicates that said patient will be responsive to an anthracycline treatment. In an alternative aspect, the genes OSM, SEMA3B, and VAV1 (together the 3-gene signature) can be used.

The present invention thus provides for methods of classifying breast cancers or stratifying breast cancer patients into subgroups of specific types of breast cancer, based on their methylation profile correlated with anthracycline treatment outcome. Based on this classification or stratification, the treatment of the cancer can be adapted, or the prognosis can be predicted.

The term "sample" or "test sample" refers to biological material obtained from a patient, and may be any tissue sample, body fluid, body fluid precipitate, or a lavage specimen. Suitable test samples for use in the methods described herein can be obtained from a breast sample, ducts or lobules of the breast tissue, a lymph node sample, or the like. The test sample may also be derived from a biological fluid, for example, whole blood, blood, lymph fluid, serum, plasma, nipple aspirate, ductal fluid, and tumor exudate. Any suitable test sample in which hypermethylation of the relevant gene(s) can be determined is included within the scope of the invention. Typical examples of test samples for use herein involve biopsies from the breast, such as conventional or needle biopsy samples. Other exemplary test samples comprise serum withdrawal or fluid samples obtained from the breast e.g. by nipple aspiration of the milk ducts or by ductal lavage of at least one breast milk duct. Nipple aspiration of breast ductal fluid is achieved by using vacuum pressure. The method may involve administering oxytocin or an oxytocin analog to a mammalian patient in an amount that is effective to stimulate expression of mammary fluid from a nipple of the patient (Suijkerbuijk et al, Ann Oncol. 2007; 18(10): 1743-1744). By the procedure of ductal lavage, ductal epithelial cells that line the walls of the ductal lumen are washed out of the duct. Lavage or wash fluid is infused into the duct, and the lavage fluid mixed with ductal fluid is collected. In embodiments, the test sample comprises a biopsy sample, preferably a needle biopsy sample, of the primary breast tumor. It has been shown in the literature that cancer or tumor cells often release genomic DNA in circulating or other bodily fluids. Since said genomic DNA has the same methylation signature of the DNA inside the tumor or cancer cell, said methylation signature can be detected in said circulating or other bodily fluid sample. This has for example been reviewed by Qureshi et al., 2010 (Int. J. Surgery 2010, 8:194-198), hereby incorporated by reference in its entirety.

A test sample can be obtained from a subject in any way typically used in clinical settings for obtaining a sample comprising the required cell or nucleic acid, including RNA, genomic DNA, mitochondrial DNA, and protein-associated nucleic acids. For example, the test sample can be obtained from fresh, frozen, or paraffin embedded surgical samples or biopsies of an organ or tissue comprising the suitable cell or nucleic acid to be tested. If desired, solid materials can be mixed with a fluid or purified or amplified or otherwise treated. For examples, samples may be treated in one or more purification steps in order to increase the purity of the desired cells or nucleic acid in the sample, or they may be examined without any purification steps. Any nucleic acid specimen in purified or non-purified form obtained from such test sample can be utilized in the methods of the present invention. In embodiments, the test sample is a frozen or formalin-fixed and paraffin-embedded biopsy, preferably a needle biopsy, of the primary breast tumor.

In the methods of the present invention, genomic DNA is isolated from the test sample from the patient for methylation analysis. Genomic DNA may be isolated by any means standard in the art, including the use of commercially available kits. Briefly, wherein the DNA is encapsulated by a cellular membrane the test sample must be disrupted and lysed by enzymatic, chemical or mechanical means. The DNA solution may then be cleared of proteins and other contaminants e.g. by digestion with proteinase K. The DNA is then recovered from the solution. This may be carried out by means of a variety of methods including salting out, organic extraction or binding of the DNA to a solid phase support. The choice of method will be affected by several factors including time, expense and required quantity of DNA. Wherein the DNA is not enclosed in a membrane (e.g. circulating DNA from a blood sample) methods standard in the art for the isolation and/or purification of DNA may be employed. Such methods include the use of a protein degenerating reagent e.g. chaotropic salt e.g. guanidine hydrochloride or urea; or a detergent e.g. sodium dodecyl sulphate (SDS), cyanogen bromide. Alternative methods include but are not limited to ethanol precipitation or propanol precipitation, vacuum concentration amongst others by means of a centrifuge. The person skilled in the art may also make use of devices such as filter devices e.g. ultrafiltration, silica surfaces or membranes, magnetic particles, polystyrene particles, polystyrene surfaces, positively charged surfaces, and positively charged membranes, charged membranes, charged surfaces, charged switch membranes, charged switched surfaces. Once the nucleic acids have been extracted, the genomic double stranded DNA is used in the methylation analysis.

In the methods of the present disclosure, hypermethylation is detected in at least one, two, three, four, five, six, seven, eight, nine, ten, eleven, twelve, thirteen, fourteen, fifteen, sixteen, seventeen, eighteen, nineteen, twenty, twenty-one, twenty-two, twenty-three or twenty-four genes selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3.

In preferred aspects, hypermethylation is detected in at least the INA gene, optionally combined with one, or more of the genes selected from the group comprising: PTPRCAP, SEMA3B, KLHL6, and RASSF1 (together the 5-gene signature). In further aspects, the method may further comprise detecting hypermethylation in at least one, two, three, four, five, six, seven, eight, nine, ten, eleven, twelve, thirteen, fourteen, fifteen, sixteen, seventeen, eighteen, nineteen, twenty, or twenty-one genes selected from the group comprising or consisting of: SHANK2, FAM113B, VSX1, DRD4, CD6, OSM, BIN2, VAV1, MT3, HEM1, LCK, EDG6, ADA, CI6orf54, LAT, SH2D3C, CD3D, PPP1R16B, and ALDH1A3.

In embodiments, hypermethylation is detected in at least the genes RASSF1, SEMA3B, KLHL6, PTPRCAP, and INA (together the 5-gene signature).

In aspects, the detection of hypermethylation of at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, comprises:
a. determining the methylation status of said at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3 in a test sample from the patient; and
b. comparing the methylation status of said at least one gene obtained in step a) to the methylation status of the same said at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3 in a control sample;
wherein an increase in methylation status indicates that said at least one gene is hypermethylated. Preferably, at least the INA gene is tested, optionally combined with one, or more of the genes selected from the group comprising: PTPRCAP, SEMA3B, KLHL6, and RASSF1 (together the 5-gene signature).

The control sample is preferably a test sample from a non-responder to anthracycline treatment.

The increase in methylation status in the test sample from the patient is preferably at least 20%, such as at least 25%, at least 30%, at least 35%, at least 40%, at least 50%; at least 60%, at least 70%, or at least 80%, compared to the methylation status in the control sample.

The detection of hypermethylation of the genes RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, may comprise:
a. determining the methylation status of said genes RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, in a test sample from the patient; and
b. calculating a signature score of said genes;
wherein a signature score higher than -0.4551 indicates that said genes are hypermethylated.

The detection of hypermethylation of the INA gene, may comprise:
a. determining the methylation status of said gene, in a test sample from the patient; and
b. calculating a signature scores of said gene;
wherein a signature score lower than -0.8555 indicates that said gene are hypermethylated.

The detection of hypermethylation of the genes RASSF1, SEMA3B, KLHL6, PTPRCAP and INA (together the 5-gene signature), may comprise:
a. determining the methylation status of said genes, in a test sample from the patient; and
b. calculating a signature scores of said genes;
wherein a signature score lower than -0.8555 indicates that said genes are hypermethylated.

The detection of hypermethylation of the genes OSM, SEMA3B, and VAV1, may comprise:
a. determining the methylation status of said genes OSM, SEMA3B, and VAV1 (together the 3-gene signature), in a test sample from the patient; and
b. calculating a signature scores of said genes;
wherein a signature score lower than -0.8555 indicates that said genes are hypermethylated.

The signature score can be computed by scaled Principal Component analysis. Briefly, the mean and the standard deviation for each gene, gene region or CpG in the signature is computed independently. Then, each beta value is scaled by subtraction of the appropriate mean and division by the appropriate standard deviation. Subsequently, a classical principal component analysis is applied by computing the Eigenvectors of the covariance matrix of the scaled values. Finally, the dot product of the scaled data with the first Eigenvector is computed.

Further disclosed herein is a method for establishing a reference methylation signature comprising determining the methylation status of at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3 in a test sample from a responder or non-responder to anthracycline treatment, thereby producing a reference methylation signature of a responder or non-responder to anthracycline treatment.

In preferred aspects, the method for establishing a reference methylation signature comprises determining the methylation status of at least the INA gene, optionally in combination with the genes selected from the group comprising: RASSF1, SEMA3B, KLHL6, and PTPRCAP (together the 5-gene signature) in a test sample from a responder or non-responder to anthracycline treatment, thereby producing a reference methylation signature of a responder or non-responder to anthracycline treatment.

In more preferred embodiments, the method for establishing a reference methylation signature comprises determining the methylation status of the genes selected from the group comprising: INA, RASSF1, SEMA3B, KLHL6, and PTPRCAP (together the 5-gene signature) in a test sample from a responder or non-responder to anthracycline treatment, thereby producing a reference methylation signature of a responder or non-responder to anthracycline treatment.

In further aspects, the method for establishing a reference methylation signature comprises determining the methylation status of at least the genes OSM, SEMA3B, and VAV1 (together the 3-gene signature) in a test sample from a responder or non-responder to anthracycline treatment, thereby producing a reference methylation signature of a responder or non-responder to anthracycline treatment. In further aspects, the method further comprises determining the methylation status of at least one, two, three, four, five, six, seven, eight, nine, ten, eleven, twelve, thirteen, fourteen, fifteen, sixteen, seventeen, eighteen, nineteen, twenty, or twenty-one genes selected from the group comprising or consisting of: RASSF1, SHANK2, FAM113B, VSX1, DRD4, CD6, BIN2, MT3, HEM1, LCK, EDG6, KLHL6, ADA, PTPRCAP, CI6orf54, INA, LAT, SH2D3C, CD3D, PPP1R16B, and ALDH1A.

In aspects, the method for establishing a reference methylation signature comprises determining the methylation status of at least the genes RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3

Also disclosed herein are reference methylation signatures obtainable by the methods described herein, as well as the use of said reference methylation signatures for the stratification of breast cancer, or the prediction of anthracycline treatment response.

Determining the methylation status of at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, comprises determining the methylation status of one or more regions of said at least one gene, preferably one or more regions as defined by SEQ ID Nos 1-29.

Accordingly, in preferred aspects of the methods of the present invention, hypermethylation is detected in at least one or more regions of the INA gene, preferably in at least the region as defined by SEQ ID No 23.

Accordingly, in preferred aspects of the methods of the present invention, hypermethylation is detected in at least one or more regions of one or more, or all of the genes selected from the group comprising: INA, RASSF1, SEMA3B, KLHL6, and PTPRCAP, preferably in at least the regions as defined by SEQ ID No 23, SEQ ID No 1 or 8, or 21, SEQ ID No 11, SEQ ID No 18, and/or SEQ ID No 12.

Accordingly, in preferred aspects of the methods of the present invention, hypermethylation is detected in at least one or more regions of the genes OSM, SEMA3B, and VAV1, preferably in at least the regions as defined by SEQ ID No 7, SEQ ID No 11, and SEQ ID No 12.

In further aspects, hypermethylation is further detected in at least one, two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, or twenty-six, gene regions as defined by SEQ ID Nos 1-6, 8-10, 13-29.

In aspects, hypermethylation is detected in at least the gene regions as defined by SEQ ID Nos 1-29.

Determining the methylation status of at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, or determining the methylation status of at least one gene region as defined by SEQ ID Nos 1-29, comprises determining the methylation status of one or more CpG dinucleotides in said at least one gene or gene region. Preferred target CpG dinucleotides according to the present invention are listed in Tables 2, 3, and 4 by the chromosal positition of the cytosine residue.

Accordingly, in preferred aspects of the methods of the present invention, the presence of at least the methylated CpG dinucleotides having a chromosomal position as defined in Table 3 or 4 is detected. In further aspects of the methods of the present invention, the presence of at least one, two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, or twenty-sixmethylated CpG dinucleotides having a chromosomal position as defined in Table 2 is further detected.

In aspects the presence of at least the methylated CpG dinucleotides having a chromosomal position as defined in Table 2 is detected.

The methylated CpG dinucleotides may be located in a coding region, such as the 5' and 3' untranslated regions, or an exon, as well as in a non-coding region, including a regulatory region such as the promoter region, of the gene. The methylated CpG dinucleotides may be located inside or outside a CpG island.

As used herein, a "methylated nucleic acid molecule" refers to a nucleic acid molecule that contains one or more nucleotides that are methylated. A gene (region) comprising at least one methylated nucleotide, preferably a methylated CpG dinucleotide, can be considered methylated (i.e. the methylation state of the gene (region) is methylated). A gene (region) that does not comprise any methylated nucleotides can be considered unmethylated.

As used herein, a "methylated nucleotide" or a "methylated nucleotide base" refers to the presence of a methyl moiety on a nucleotide base, where the methyl moiety is not present in a recognized typical nucleotide base. Cytosine does not contain a methyl moiety on its pyrimidine ring, however 5-methylcytosine contains a methyl moiety at position 5 of its pyrimidine ring. In this respect, cytosine is not a methylated nucleotide and 5-methylcytosine is a methylated nucleotide.

As used herein, a "methylated CpG dinucleotide" refers to the presence of a methyl moiety on the cytosine nucleotide of the CpG dinucleotide, i.e. the presence of 5-mehtylcytosine in a CpG dinucleotide.

As used herein "CpG dinucleotide" refers to a nucleic acid region where a cytosine nucleotide occurs next to a guanine nucleotide in the linear sequence of bases along its length.

As used herein, a "methylation site" is a nucleotide within a nucleic acid that is susceptible to methylation either by natural occurring events *in vivo* or by an event instituted to methylate the nucleotide *in vitro.*

The term "regulatory region" or the equivalent "regulatory sequence," in respect of a specific gene, refers to a region of the non-coding nucleotide sequences within that gene that are necessary or sufficient to provide for the regulated expression of the coding region of said gene. Thus, the term "regulatory region" includes promoter sequences, regulatory protein binding sites, upstream activator sequences, and the like. Specific nucleotides within a regulatory region may serve multiple functions. For example, a specific nucleotide may be part of a promoter and participate in the binding of a transcriptional activator protein.

"Promoter" refers to a nucleotide sequence capable of controlling the expression of a coding sequence or functional RNA. In general, a coding sequence is located 3' to a promoter sequence. The promoter sequence consists of proximal and more distal upstream elements, the latter elements often referred to as enhancers. Accordingly, an "enhancer" is a nucleotide sequence which can stimulate promoter activity and may be an innate element of the promoter or a heterologous element inserted to enhance the level or tissue-specificity of a promoter. It is understood by those skilled in the art that different promoters may direct the expression of a gene in different tissues or cell types, or at different stages of development, or in response to different environmental conditions. Typically, a "promoter" region extends between approximately 1 Kb, 500 bp or 150 to 300 bp upstream from the transcription start site.

The term "CpG island" is a G:C-rich region of genomic DNA containing a greater number of CpG dinucleotides relative to total genomic DNA, as defined in the art. In many genes, the CpG islands begin just upstream of a promoter and extend downstream into the transcribed region. The islands can also surround the 5' region of the coding region of the gene as well as the 3' region of the coding region. CpG islands can be found in multiple regions of a gene like upstream of coding regions in a regulatory region including a promoter region; within the coding regions (e.g., exons); downstream of coding regions in, for example, enhancer regions; or within introns. All of these regions can be assessed to determine their methylation status. It should be noted that hypermethylation of the target genes according to the invention is not limited to CpG islands only, but can be in so-called "shores" or "shelves" or can be lying completely outside a CpG island region.

The term "methylation status" or "methylation state" or "methylation profile," when used herein to describe the state of methylation of a gene (region) refers to the characteristics of said gene (region) at a particular locus relevant to methylation, i.e. at particular CpG dinucleotides. Such characteristics include, but are not limited to, whether any of the cytosine (C) residues within this gene (region) are methylated, location of methylated C residue(s), ratio or percentage of methylated C at any particular stretch of residues, and allelic differences in methylation due to, e.g., difference in the origin of the alleles. The term "methylation profile" or "methylation state" or "methylation status" also refers to the relative or absolute concentration of methylated C or unmethylated C at any particular stretch of residues.

By "aberrant methylation" is meant altered methylation of cytosine (C) residues of CpG dinucleotides in a gene (region), which are converted to 5'-methylcytosine. Such altered methylation is measured in comparison to the same regions of the gene (region) in a control sample. Two forms of aberrant methylation are possible: hypermethylation and hypomethylation.

The term "hypermethylation," when used in reference to a gene (region) means that the characteristics relevant to methylation as describe above are found in a relative abundance; for example the cytosine residues of CpG dinucleotides associated with the gene (region) are methylated in a relative abundance at the CpG-position. The methylation state or status of gene (region) may be expressed as a percentage of methylatable nucleotides (e.g., cytosine) or CpG dinucleoties in a gene (region) that are methylated (e.g., about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% or about 100% methylated; greater than 80% methylated, between 20% to 80% methylated, or less than 20% methylated). Accordingly, a nucleic acid, in particular a gene (region), may be "hypermethylated," which refers to the nucleic acid, in particular the gene (region), having a greater number of methylatable nucleotides or CpG dinucleotides that are methylated relative to a control or reference. A nucleic acid, in particular a gene (region), may be "hypomethylated," which refers to the nucleic acid, in particular the gene (region), having a smaller number of methylatable nucleotides or CpG dinucleotides that are methylated relative to a control or reference.

As used herein, a "methylation profile" or "methylation state" or "methylation status" correlated with a treatment outcome refers to a specific methylation state of a gene (region) or CpG dinucleotide that is present or absent more frequently in subjects with a specific treatment outcome, relative to the methylation state of a gene (region) or CpG site than otherwise occur in a larger population of individuals (e.g., a population of all individuals).

As used herein, "methylation ratio" refers to the number of instances in which a molecule or locus is methylated relative to the number of instances the molecule or locus is unmethylated. Methylation ratio can be established regarding a population of individuals or in respect of a sample from a single individual. For example, a CpG dinucleotide having a methylation ratio of 50% is methylated in 50% of instances and unmethylated in 50% of instances. Such a ratio can be used, for example, to describe the degree to which a molecule or locus is methylated in a population of individuals. Thus, when methylation in a first population or pool of nucleic acid molecules is different from methylation in a second population or pool of nucleic acid molecules, the methylation ratio of the first population or pool will be different from the methylation ratio of the second population or pool. Alternatively such a ratio can be used to describe the degree to which a nucleotide locus or nucleic acid region is methylated in a single individual. For example, such a ratio can be used to describe the degree to which a gene (region) of a group of cells from a test sample, are methylated or unmethylated at a nucleotide locus or methylation site, in particular a CpG dinucleotide.

The methylation status of a gene, a gene region, or a CpG dinucleotide as described herein may be determined by any technique for detecting methylated nucleic acid molecules. These techniques are based on any one or more of the classic techniques of hybridization, amplification, sequencing, electrophoresis, chromatography, and mass spectrometry and combinations thereof, amongst others, and include, for example, but without limitation, the polymerase chain reaction (PCR), methylated CpG island recovery assay (MIRA), combined bisulfite-restriction analysis (COBRA), MALDI-TOFF, MassARRAY, MethyLight, Quantitative analysis of methylated alleles (QAMA), Enzymatic regional methylation assay (ERMA), HeavyMethyl, QBSUPT, MS-SNuPE, MethylQuant, Quantitative PCR sequencing, (bisulfite) pyrosequencing, single-strand conformation polymorphism (SSCP) analysis, restriction analysis, bead-chip technology, ligase chain reaction, microarray analysis, oligonucleotide-based microarray, amongst other.

Methylation specific PCR (US 5,786,146, US 6,017,704, US 6,200,756) allows the distinction between modified and unmodified DNA by hybridizing oligonucleotide primers which specifically bind to one form or the other of the DNA. After hybridization, an amplification reaction can be performed and amplification products assayed. The presence of an amplification product indicates that a sample hybridized to the primer. The specificity of the primer indicates whether the DNA had been modified or not, which in turn indicates whether the DNA had been methylated or not. For example, bisulfite ions modify non-methylated cytosine bases, changing them to uracil bases. Uracil bases hybridize to adenine bases under hybridization conditions. Thus an oligonucleotide primer which comprises adenine bases in place of guanine bases would hybridize to the bisulfite-modified DNA, whereas an oligonucleotide primer containing the guanine bases would hybridize to the non-modified (methylated) cytosine residues in the DNA. Amplification using a DNA polymerase and a second primer yield amplification products that can be readily observed. The amplification products can be optionally hybridized to specific oligonucleotide probes which may also be specific for certain products. Alternatively, oligonucleotide probes can be used which will hybridize to amplification products from both modified and non-modified DNA.

Any reference to "modified" bases or sequences, obviously in the appropriate context, alludes to the treatment of these bases or sequences with a suitable reagent, in order to convert non-methylated cytosine into uracil bases.

With real-time methylation specific PCR, the methylation profile of the genes of interest can be assessed by determining the amplification level of said genes based on amplification-mediated displacement or on binding of one or more probes whose binding sites are located within the amplicon. In general, real-time quantitative methylation specific PCR is based on the continuous monitoring of a progressive fluorogenic PCR by an optical system. Such PCR systems usually use two amplification primers and an additional amplicon-specific, fluorogenic hybridization probe that specifically binds to a site within the amplicon. The probe can include one or more fluorescence label moieties. For example, the probe can be labeled with two fluorescent dyes: 1) a 6-carboxy-fluorescein (FAM), located at the 5'-end, which serves as reporter, and 2) a 6-carboxy-tetramethyl-rhodamine (TAMRA), located at the 3'-end, which serves as a quencher. When amplification occurs, the 5'-3' exonuclease activity of the Taq DNA polymerase cleaves the reporter from the probe during the extension phase, thus releasing it from the quencher. The resulting increase in fluorescence emission of the reporter dye is monitored during the PCR process and represents the number of DNA fragments generated. This process is known as Taqman. Other systems to monitor real-time PCR involve the use of hairpin primers (Amplifluor), hairpin probes (Molecular Beacons), FRET probe pairs (Lightcycler), primers incorporating a hairpin probe (Scorpion), Plexor™ system, primers incorporating complementary sequences of DNAzymes that cleave a reporter substrate included in the reaction mixture (DzyNA) or fluorescent dyes (SYBR Green etc.)

Methylation-dependent sequence variation at CpG dinucleotide motifs offers different approaches to PCR primer design. In one approach, oligonucleotide primers are designed to specifically bind methylated primer-binding sites, and a probe is designed to anneal specifically within the amplicon during extension. Such primers typically comprise CpG dinucleotides which get affected by DNA methylation. In another approach, oligonucleotide primers are designed to bind either methylated or unmethylated primer-binding sites, and the probe is designed to anneal specifically to methylated probe binding sites. In yet another approach, both oligonucleotide primers and probes are designed to specifically bind methylated binding sites. In either approach, when there is a sufficient region of complementarity, e.g., 12, 15, 18, or 20 nucleotides, between the target and the primer, the primer may also contain additional nucleotide residues that do not interfere with hybridization but may be useful for other manipulations. Exemplary of such other residues may be sites for restriction endonuclease cleavage, for ligand binding, or for factor binding or linkers or repeats.

Another way to distinguish between modified and non-modified DNA employs oligonucleotide probes which may also be specific for certain products. Such probes can be hybridized directly to modified DNA or to amplification products of modified DNA. Oligonucleotide probes can be labeled using any detection system known in the art. These include but are not limited to fluorescent moieties, radioisotope labeled moieties, bioluminescent moieties, luminescent moieties, chemiluminescent moieties, enzymes, substrates, receptors, or ligands. The oligonucleotide probes may be bound to a support. The support can be any suitable support such as plastic materials, (fluorescent) beads, magnetic beads, synthetic or natural membranes, latex beads, polystyrene, column supports, glass beads or slides, nanotubes, fibres or other organic or inorganic supports. The binding processes are well-known in the art and generally comprise cross-linking, covalently binding or physically adsorbing the oligonucleotide to the support. A support may also contain a plurality of oligonucleotide probes arrayed on the support. Such array may comprise multiple copies of the same oligonucleotide probe so as to capture the same target gene (region) on the array or may comprise a plurality of different oligonucleotide probes targeting different genes (regions) so as to capture a plurality of target genes (regions) on the array. An exemplary method for detecting the methylation status of a nucleic acid based on the use of oligonucleotide probes is the use of Infinium® BeadChip sold by Illumina Inc. San Diego (US), which makes use of a bead chip.

Another approach for assessing the methylation status of a gene (region) uses methylation-sensitive restriction endonucleases to detect methylated CpG dinucleotide motifs. Such endonucleases may either preferentially cleave methylated recognition sites relative to non-methylated recognition sites, or preferentially cleave non-methylated relative to methylated recognition sites. Examples of the former are Acc III, Ban I, BstN I, Msp I, and Xma I. Examples of the latter are Acc II, Ava I, BssH II, BstU I, Hpa II, and Not I. Yet another approach is the combined bisulfite-restriction analysis (COBRA assay) in which PCR products obtained from bisulfite-treated DNA are further analyzed by using restriction enzymes that recognize sequences containing 5'CG, such as Taql (5'TCGA) or BstUI (5'CGCG), such that methylated and unmethylated DNA can be distinguished.

Following digestion of sample DNA with either methylation-sensitive or methylation-insensitive restriction enzymes (for ex. Mspl and Hpall), the DNA can be analyzed by methods such as Southern Blotting and PCR. Southern blot analysis involves electrophoretic separation of the resulting DNA fragments and hybridization with a labeled probe adjacent to the CpG of interest. If the hybridization signal from the methylation-sensitive and methylation-insensitive digested DNA samples results in different size bands, than the site of interest was methylated. In contrast, PCR analysis involves amplification across the CpG of interest. The expected band will only be observed in the methylation-sensitive digested sample if the site of interest is methylated. The PCR assay requires much lower amounts of DNA for each site of interest (for ex: 1-10 ng), but necessitates the design and testing of specific primer pairs for every site of interest.

Still another way for the identification of methylated CpG dinucleotides utilizes the ability of the MBD domain of the MeCP2 protein to selectively bind to methylated DNA sequences (Cross et al, 1994; Shiraishi et al, 1999). Restriction endonuclease digested genomic DNA is loaded onto expressed His-tagged methyl-CpG binding domain that is immobilized onto a solid matrix and used for preparative column chromatography to isolate highly methylated DNA sequences.

Alternatively, the HELP assay can be used, which is based on the differential ability of restriction enzymes to recognize and cleave methylated and unmethylated CpG DNA sites.

Furthermore, ChIP-on-chip assays, based on the ability of commercially prepared antibodies to bind to DNA methylation-associated proteins like MCP2, can be used to determine the methylation status. Also restriction landmark genomic scanning, also based upon differential recognition of methylated and unmethylated CpG sites by restriction enzymes can be used. Methylated DNA immunoprecipitation (MeDIP), analogous to chromatin immunoprecipitation, can be used to isolate methylated DNA fragments for input into DNA detection methods such as DNA microarrays (MeDIP-chip) or DNA sequencing (MeDIP-seq). The unmethylated DNA is not precipitated. Alternativley, molecular break light assay for DNA adenine methyltransferase activity can be used. This is an assay that uses the specificity of the restriction enzyme Dpnl for fully methylated (adenine methylation) GATC sites in an oligonucleotide labeled with a fluorophore and quencher. The adenine methyltransferase methylates the oligonucleotide making it a substrate for Dpnl. Cutting of the oligonucleotide by Dpnl gives rise to a fluorescence increase. Further, methylated-CpG island recovery assay (MIRA) can be used.

Typically, for determining the methylation status of a gene, a region thereof, or a CpG dinucleotide thereof, genomic DNA or a fragment thereof, is treated with a reagent that selectively modifies an unmethylated cytosine residue but which is incapable of modifying a methylated cytosine residue; and the resulting product is detected. Examples of reagents for selective modification of unmethylated cytosine residues include hydrazine and bisulfite ions. Hydrazine-modified DNA, or a portion thereof, can be treated with piperidine to cleave it. Bisulfite ion-treated DNA, or a portion thereof, can be treated with alkali. The resulting products can be detected directly, or after a further reaction that creates products, which are easily distinguishable. For example, the products resulting from the treatment with a reagent that selectively modifies an unmethylated cytosine residue but which is incapable of modifying methylated cytosine residue, may be detected by amplification with at least one primer that hybridizes to a sequence comprising a modified non-methylated CpG dinucleotide motif, i.e. UpG or TpG, but not to a sequence comprising an unmodified methylated CpG dinucleotide motif, thereby forming amplification products. Conversely, the resulting products may be detected by amplification with at least one primer that hybridizes to a sequence comprising an unmodified methylated CpG dinucleotide motif but not to a sequence comprising a modified non-methylated CpG, i.e. UpG or TpG, dinucleotide motif, thereby forming amplification products. In addition, the amplification products under investigation may be detected using (a) a first oligonucleotide probe which hybridizes to a sequence comprising a modified non-methylated CpG dinucleotide motif, i.e. UpG or TpG, but not to a sequence comprising an unmodified methylated CpG dinucleotide motif, (b) a second oligonucleotide probe that hybridizes to a sequence comprising an unmodified methylated CpG dinucleotide motif but not to a sequence comprising a modified non-methylated CpG dinucleotide motif, i.e. UpG or TpG, or (c) both said first and second oligonucleotide probes.

Accordingly, in embodiments of the methods described herein, determining the methylation status of a gene, a gene region, or a CpG dinucleotide as described herein comprises:
a) isolating genomic DNA from a test sample from the patient;
b) contacting the genomic DNA obtained in step a) with at least one reagent that selectively modifies an unmethylated cytosine residue but which is incapable of modifying a methylated cytosine residue;
c) detecting the modified DNA obtained in step b) with a technique as described above.

The methods of the present invention may be used for diverse diagnostic, clinical, toxicological, research, and forensic purposes including, drug discovery, designing patient therapy, drug efficacy testing, and patient management. The present methods may be used in combination with other assays. The results may be implemented in computer models and databases.

The present disclosure further relates to the use of the hypermethylation at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, preferably at least the INA gene, optionally in combination with the genes selected from the group comprising: RASSF1, SEMA3B, KLHL6, and PTPRCAP (together the 5-gene signature), as a marker or preclinical marker, of anthracycline treatment response in a breast cancer patient. In an alternative aspect, the genes OSM, SEMA3B, and VAV1 (together the 3-gene signature) can be used.

Also described herein is the use of the hypermethylation at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, preferably at least the INA gene, optionally in combination with the genes selected from the group comprising: RASSF1, SEMA3B, KLHL6, and PTPRCAP (together the 5-gene signature)as a preclinical marker of anthracycline treatment response in a breast cancer patient. In an alternative aspect the genes OSM, SEMA3B, and VAV1 (together the 3-gene signature) can be used.

The term "marker" is synonym to "biomarker" or "biological marker" in the present invention.

A "biomarker," as defined by the National Institutes of Health (NIH) is a molecular indicator of a specific biological property; a biochemical feature or facet that can be used to measure the progress of disease or the effects of treatment. Thus, a biomarker is an organic biomolecule which is differentially present in a sample taken from a subject of one phenotypic status (e.g., having a disease) as compared with another phenotypic status (e.g., not having the disease). It is differentially present between different phenotypic statuses if the mean or median expression level of the biomarker in the different groups is calculated to be statistically significant. Common tests for statistical significance include, among others, t-test, ANOVA, Kruskal-Wallis, Wilcoxon, Mann-Whitney and odds ratio. Biomarkers, alone or in combination, provide measures of relative risk that a subject belongs to one phenotypic status or another. Therefore, they are useful inter alia, as markers for disease (diagnostics), therapeutic effectiveness of a drug (theranostics) toxicity. Based on the invention detailed herein, differential methylation of the biomarkers in a test sample from a patient relative to a control sample makes it possible to predict anthracycline treatment outcome.

A "biological marker" is defined by MESH (the Medical Subject Headings of the National Library of Medicine Controlled Vocabulary) as a measurable and quantifiable biological parameter (e.g., specific enzyme concentration, specific hormone concentration, specific gene phenotype distribution in a population, presence of biological substances) which serves as indices for health- and physiology-related assessments, such as disease risk, psychiatric disorders, environmental exposure and its effects, disease diagnosis, metabolic processes, substance abuse, pregnancy, cell line development, epidemiologic studies, etc.

The term "preclinical marker" refers to a biomarker as defined above, which has not yet been validated in a clinical trial.

The methylation status of the differentially methylated CpG dinucleotides of the gene (region) of interest can provide a variety of information about the disease of breast cancer. It can be used to assess intratumoral lymphocyte infiltration (TIL). It can be used to diagnose breast (pre)cancer in the individual. Alternatively, it can be used to predict the course of the disease in the individual or to predict the susceptibility to the disease, or to stage the progression of the disease in the individual. Importantly, it can help to predict the likelihood of pCR, overall survival, cause-specific survival, disease-free survival, or predict the likelihood of reoccurrence of breast cancer, and determine the effectiveness of a treatment course undergone by the individual. In particular, it can determine the effectiveness of an anthracycline treatment to be undergone by an individual. Increase or decrease of methylation levels in comparison with a reference level, and alterations in the increased/decreased detected levels provide valuable prognostic and diagnostic information.

Also described herein is an anthracycline compound for use in the prevention or treatment of a breast cancer patient, characterised in that said patient has hypermethylation in at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, preferably at least the INA gene, optionally in combination with the genes selected from the group comprising: RASSF1, SEMA3B, KLHL6, and PTPRCAP (together the 5-gene signature). In an alternative aspect, the genes OSM, SEMA3B, and VAV1 (together the 3-gene signature) can be used.

Also described herein is method of prevention or treatment of a patient suffering from breast cancer comprising administering to said patient an effective amount of an anthracycline compound, characterised in that said patient has hypermethylation in at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, preferably at least the INA gene, optionally in combination with the genes selected from the group comprising: RASSF1, SEMA3B, KLHL6, and PTPRCAP (together the 5-gene signature). In an alternative aspect, the genes OSM, SEMA3B, and VAV1 (together the 3-gene signature) can be used.

Said anthracycline compound may be administered alone or in a combination regimen as known in the art.

As used herein, a "nucleic acid target" is a nucleic acid molecule that is examined using the methods disclosed herein. For the purposes of the application, "nucleic acid target", "target gene", "target" may be used interchangeably. A nucleic acid target includes genomic DNA or a fragment thereof, which may or may not be part of a gene, a segment of mitochondrial DNA of a gene or RNA of a gene and a segment of RNA of a gene. As used herein, a "nucleic acid target molecule" is a molecule comprising a nucleic acid sequence of the nucleic acid target. The nucleic acid target molecule may contain less than 10%, less than 20%, less than 30%, less than 40%, less than 50%, greater than 50%, greater than 60%, greater than 70% greater than 80%, greater than 90% or up to 100% of the sequence of the nucleic acid target.

The disclosure further relates to a set of oligonucleotides for determining the methylation status of at least one gene selected from the group comprising or consisting of: RASSF1, SHANK2, FAM113B, VSX1, DRD4, CD6, OSM, BIN2, SEMA3B, VAV1, MT3, HEM1, LCK, EDG6, KLHL6, ADA, PTPRCAP, CI6orf54, INA, LAT, SH2D3C, CD3D, PPP1R16B, and ALDH1A3, or at least one gene region as defined by SEQ ID Nos 1-29, or at least one CpG dinucleotide having a chromosomal position as defined in Table 2, said set of oligonucleotides comprising or consisting of isolated oligonucleotides comprising a nucleotide sequence complementary to SEQ ID Nos 1-29.

In preferred aspects, the set of oligonucleotides comprises or consists of isolated oligonucleotides comprising a nucleotide sequence complementary to SEQ ID No 23. In further aspects, the set of oligonucleotides further comprises one or more isolated oligonucleotides comprising a nucleotide sequence complementary to SEQ ID Nos 1, 8, 21, 11, 18, or 12.

In preferred aspects, the set of oligonucleotides comprises or consists of isolated oligonucleotides comprising a nucleotide sequence complementary to SEQ ID No 7, SEQ ID No 11, and SEQ ID No 12. In further aspects, the set of oligonucleotides further comprises one or more isolated oligonucleotides comprising a nucleotide sequence complementary to SEQ ID Nos 1-6, 8-10, 13-29.

In aspects, the set of oligonucleotides comprises or consists of isolated oligonucleotides comprising a nucleotide sequence complementary to SEQ ID Nos 1-29.

The invention further relates to the use of the sets of oligonucleotides described herein for determining an anthracycline treatment outcome in a patient with breast cancer; for predicting susceptibility to an anthracycline treatment in a patient with breast cancer; for the stratification of breast cancer patients; or for selecting a suitable treatment for breast cancer in a patient.

Also disclosed herein is a kit for the stratification of breast cancer or the prediction of response to anthracycline treatment, comprising the set of oligonucleotides described herein. The kit may further comprise a reagent that selectively modifies an unmethylated cytosine residue but which is incapable of modifying a methylated cytosine residue. The kit may also comprise one or more reference methylation signatures according to the present invention, and/or means for determining the methylation status.

Also described herein is the use of the kits described herein for determining an anthracycline treatment outcome in a patient with breast cancer; for predicting susceptibility to an anthracycline treatment in a patient with breast cancer; for the stratification of breast cancer patients; or for selecting a suitable treatment for breast cancer in a patient.

The following non-limiting examples help to illustrate the principles of the invention.

### Examples

### Materials and methods

### Patients

### - Study population

TOP cohort: The neoadjuvant prospective "TOP" (Trial of Principle) trial was conducted at different European hospitals and coordinated by the Institut Jules Bordet. This study is registered on the clinical trials site of the US National Cancer Institute website http://clinicaltriats.gov/ct2/show/NCT00162812?term=NCT00162812&rank=1. One hundred and forty-nine patients have been included in this trial. Anthracycline (epirubicin) monotherapy (100 mg/m2) was given as neo-adjuvant chemotherapy: every 3 weeks x 4 cycles for early BC or every 2 weeks x 6 cycles with Neulasta® 6 mg on day 2 for patients with inflammatory or locally advanced breast cancer. At completion of chemotherapy, every patient underwent surgery with axillary node sampling. After surgery, adjuvant docetaxel (100 mg/m2 x 4 cycles) and loco-regional irradiation were administered using standard criteria. This study was primarily designed for the identification of biomarkers of response to anthracyclines (epirubicin).

All patients underwent pretreatment core biopsies of the primary breast tumor before starting neo-adjuvant chemotherapy using a 14G needle. Two biopsies were embedded in OCT (Sakura), frozen in liquid nitrogen within 5 minutes and transferred to a -80°C freezer. Two biopsies were fixed in formalin and embedded in paraffin. Both fixed and frozen samples were retrieved and stored at the Institut Jules Bordet in Brussels, where the TOP2A evaluations were carried out. Pathologic response was determined by microscopic examination of the excised tumor and nodes after completion of chemotherapy. Pathological complete response (pCR) was defined by the absence of residual invasive breast carcinoma (macro and microscopic) in the breast and in the axillary nodes. Persistence of in-situ carcinoma without invasive component was considered pCR.

This analysis was performed after the clinical data until surgery had become available for all the patients. The clinical data was collected, monitored and validated by the BrEAST data centre, Institut Jules Bordet. This study has been approved by the local ethics committees and all patients have given written informed consent prior to study entry.

### - Further patient cohorts

The PNC1 breast cancer cohort is constituted of 119 archival frozen breast cancer samples from patients diagnosed at the Jules Bordet Institute in Brussels between 1995 and 2003. These samples were selected according to the following criteria:
1/ sufficient presence of invasive cells as defined by pathologist. The current practice of pathologists is to examine by microscopy a representative slide of a given tumour sample and to estimate the proportion of the tumour that contains epithelial cancer cells (measured as « % area »). Any sample below an arbitrary threshold of an estimated value of "90%" was rejected. Although this is a current practice of pathologists and has been for many years, it is important to notice that this "area" criterion is not quantitatively accurate;
2/ >2 µg yield of high quality DNA available;
3/ balanced distribution of the four main "breast cancer expression subtypes" determined by immunohistochemistry; and
4/ balanced distribution of patients with and without relapses within each subtype.

Four samples of normal breast tissues with sufficient high-quality DNA were selected as well for this validation cohort.

Furthermore, a discovery cohort (cohort 1) was used comprising 105 archival frozen breast cancer samples from patients and a validation cohort (cohort 2) was used comprising 115 archival frozen breast cancer samples from patients, to discover and validate the 5-gene signature as discussed herein. This 5-gene signature was verified with an independent TCGA (The Cancer Gene Atlas) cohort.

### Cell lines and culture

MCF10A cells were cultured in DMEM/F12 (1:1) medium (Gibco); MCF-7, SKBR3 and MDAMB-231 were cultured in DMEM medium (Gibco); T47D, ZR-75-1 and MDAMB-361 were cultured in RPMI medium (Gibco); and BT20 were cultured in MEM medium (Gibco). For all breast epithelial cell lines, media were supplemented with 10% fetal calf serum (Gibco).

The lymphoid clones CD4+ R12C9 and CD8+ WEIS3E5 were maintained in Isocove Dubelcco medium supplemented with 10% human serum HS54, L Arginine, L-Asparagine, L-glutamine, 2-mercaptoethanol and methyltryptophane as well as with 10 ng/mL of IL-7 and 50 U/mL of IL-2.

### DNA methylation profiling

Briefly, genomic DNA from the clinical frozen samples was extracted from twenty 10-µm sections using the Qiagen-DNeasy Blood &Tissue Kit according to the supplier's instructions (Qiagen, Hilden, Germany). This included a proteinase K digestion at 55°C overnight. DNA was quantitated with the NanoDrop® ND-1000 UV-Vis Spectrophotometer (NanoDrop Technologies, Wilmington, DE, USA). Site-specific CpG methylation was analysed using Infinium® HumanMethylation27 (27k) BeadChip or Infinum®HumanMethylation450 (450k)BeadChip (Illumina, San Diego, CA, USA) in accordance with the supplier's instructions. The 27k array was developed to assay 27,578 CpG sites selected from more than 14,000 genes. The 450k platform assays over 450,000 methylation sites, thereby allowing for an almost whole-genome analysis of DNA methylation and covering 99% of the RefSeq genes. Genomic DNA (1µg) was treated with sodium bisulphite using the Zymo EZ DNA Methylation KitTM (Zymo Research, Orange, USA) according to the manufacturer's procedure. The methylation assay was performed from 4 µL converted gDNA at 50 ng/µL. The quality of bead array data was checked with the GenomeStudio® Methylation Module software. All samples passed this quality control.

### MeTIL score and optimized MeTIL score

The signature scores were computed as follows: first, the mean and the standard deviation for each CpG (for each probe) of the respective signatures (i.e. the MeTIL signature or the optimized MeTIL signature) were computed independently. Then, each beta value was scaled by subtraction of the appropriate mean and division by the appropriate standard deviation. Subsequently, a classical principal component analysis was applied by computing the Eigenvectors of the covariance matrix of the scaled values. Finally, the dot product of the scaled data with the first Eigenvector was computed.

### IR and STAT1 signatures and scores

The IR and STAT1 signatures have been described in Desmedt et al. 2008 (Clin Cancer Res 14:5158-5165), which is specifically incorporated by reference herein. The signature scores of the IR and STAT1 signatures were computed by a Principal Component Analysis (PCA).

### Example 1: Identification and validation of predictive MeTIL signatures for the response to anthracycline treatment.

Predictive DNA methylation markers for the response to anthracycline treatment were identified in two steps: 1) genes that are differently (> 80% difference in delta beta value) DNA methylated in normal T lymphocyte cell lines and epithelial breast cancer cells were identified, and 2) the identified signature was tested for prediction of response to anthracycline treatment in the TOP cohort.

DNA methylation profiles from epithelial breast cancer cell lines and normal T lymphocyte cell lines were established by the Infinium® 27k platform A signature of 29 probes corresponding to 24 individual genes that are differently DNA methylated in T lymphocytes and epithelial breast cancer cells was identified (Table 2).

**Table 2: MeTIL signature of genes that are differently (> 80% difference in delta beta value) DNA methylated in T lymphocytes and epithelial breast cancer cells. Abbreviations: RASSF1: Homo sapiens Ras association (RaIGDS/AF-6) domain family member 1: SHANK2: Homo sapiens SH3 and multiple ankyrin repeat domains 2; FAM113B: Homo sapiens PC-esterase domain containing 1B; VSX1: Homo sapiens visual system homeobox 1; DRD4: Homo sapiens dopamine receptor D4; CD6: Homo sapiens CD6 molecule; OSM: Homo sapiens oncostatin M; BIN2: Homo sapiens bridging integrator 2; SEMA3B: Homo sapiens sema domain, immunoglobulin domain, secreted, (semaphorin) 3B domain (Ig), short basic; VAV1: Homo sapiens vav 1 guanine nucleotide exchange factor; MT3: Homo sapiens metallothionein3; HEM1: Homo sapiens NCK-associated protein 1-like; LCK: Homo sapiens lymphocyte-specific protein tyrosine kinase; EDG6: Homo sapiens sphingosine-1-phosphate receptor 4; KLHL6: Homo sapiens kelch-like family member 6; ADA: Homo sapiens adenosine deaminase; PTPRCAP: Homo sapiens protein tyrosine phosphatase, receptor type, C-associated protein; CI6orf54: Homo sapiens chromosome 16 open reading frame 54; INA: Homo sapiens internexin neuronal intermediate filament protein, alpha; LAT: Homo sapiens linker for activation of T cells; SH2D3C: Homo sapiens SH2 domain containing 3C; CD3D: Homo sapiens CD3d molecule, delta (CD3-TCR complex); PPP1R168: Homo sapiens protein phosphatase 1, regulatory subunit 16B; ALDH1A3: Homo sapiens aldehyde dehydrogenase 1 family, member A3**

| **IlluminaID** | **Corresponding gene** | **Correspondin g RefSeq mRNA ID** | **Chromosomal position cytosine** | **Target sequence** |
|---|---|---|---|---|
| cg00777121 | RASSF1 | NM_007182;NM_170714 | chr3:50378191 | |
| cg04396791 | SHANK2 | NM_012309;NM_133266 | chr11:70508180 | |
| cg05596756 | FAM113B | NM_138371;NM_138371;NR_026544 | chr12:47610220 | |
| cg06151165 | VSX1 | NM_014588;NM_199425 | chr20:25062254 | |
| | | | | |
| cg06825142 | DRD4 | NM_000797 | chr11:637170 | |
| cg07380416 | CD6 | NM_006725 | chr11:60739172 | |
| cg07498879 | OSM | NM_020530 | chr22:30663041 | |
| cg08047457 | RASSF1 | NM_007182;NM_170714 | chr3:50378413 | |
| cg09902130 | CD6 | NM_006725 | chr11:60739178 | |
| cg10590292 | BIN2 | NM_016293 | chr12:51717674 | |
| cg12069309 | SEMA3B | NM_001005914;NM_004636 | chr3:50312913 | |
| cg13470920 | VAV1 | NM_005428 | chr19:6772831 | |
| cg14519350 | OSM | NM_020530 | chr22:30661949 | |
| cg16158681 | MT3 | NM_005954 | chr16:56623109 | |
| cg16509569 | HEM1 | NM_005337 | chr12:54891634 | |
| cg17078393 | LCK | NM_005356 | chr1:32717002 | |
| cg17518965 | EDG6 | NM_003775 | chr19:3178955 | |
| | | | | |
| cg20425130 | KLHL6 | NM_130446 | chr3:183273245 | |
| cg20622019 | ADA | NM_000022 | chr20:43279793 | |
| cg20792833 | PTPRCA P | NM_005608 | chr11:67205195 | |
| cg21554552 | RASSF1 | NM_007182;NM_170714 | chr3:50378425 | |
| cg23093496 | CI6orf54 | NM_175900 | chr16:29757323 | |
| cg23642747 | INA | NM_032727 | chr10:105036645 | |
| cg23797100 | LAT | NM_001014989;NM_001014987;NM_014387;NM_001014988 | chr16:28996053 | |
| cg24545967 | SH2D3C | NM_170600 | chr9:130540941 | |
| cg24841244 | CD3D | NM_001040651 ;NM_000732 | chr11:118213330 | |
| cg26285698 | CI6orf54 | NM_175900 | chr16:29757334 | |
| cg27377213 | PPP1R16 B | NM_015568 | chr20:37433803 | |
| cg27652350 | ALDH1A3 | NM_000693 | chr15:101420989 | |

The signature obtained in the first step was then tested for prediction of response to anthracycline treatment in the TOP cohort. 450k methylation assays were performed on pre-chemotherapy fresh-frozen biopsies from 58 patients from the TOP cohort, in particular 7 responders (i.e. patients showing pathologic complete response (pCR)) and 51 non-responders to anthracycline treatment.

Principal Component (PC) analysis was used to compute a first 3-gene signature score (Fig. 1), MeTIL (MeT) Score, which was used to generate a receiver operating characteristic (ROC) curve for the prediction of pCR (Fig. 2). As shown in figure 2, this signature identifies patients in the TOP cohort who do not benefit from anthracycline treatment with a specificity of 51% and a sensitivity of 100%.

This 3-gene MeTIL signature was in a subsequent step optimized for prediction of pCR in the TOP cohort using a Machine Learning approach, wherein the DNA methylation beta value of the 29 probes was regressed across response status of the TOP cohort. Briefly, a leave-one-out procedure was used combining mRMR filtering and a Support Vector Machine prediction method that simultaneously give a prediction power estimation and discard the less informative CpG. By applying iteratively this procedure, the prediction power for sets of CpG of size 29 to 1 was obtained. As final signature the set of CpG giving simultaneously the highest sensitivity and specificity was selected. This optimized signature contained 3 probes corresponding to 3 individual genes (Table 3). Again, PC analysis was used to calculate a signature score (Fig. 3), the optimized MeTIL Score, and based on that score a ROC curve was generated for the prediction of response (Fig. 4). This optimized MeTIL signature identified patients who do not benefit from anthracycline treatment with a specificity and sensitivity of 73% and 100%, respectively.

**Table 3: Optimized 3-gene MeTIL signature Abbreviations: OSM: Homo sapiens oncostatin M; SEMA3B: Homo sapiens sema domain, immunoglobulin domain, secreted, (semaphorin) 3B domain (Ig), short basic; VAV1: Homo sapiens vav 1 guanine nucleotide exchange factor.**

| **IlluminaID** | **Correspondi ng gene** | **Correspondi ng RefSeq mRNA ID** | **Chromosomal position cytosine** | **Target sequence** |
|---|---|---|---|---|
| cg07498879 | OSM | NM_020530 | chr22:30663041 | |
| cg12069309 | SEMA3B | NM_001005914;NM_0046 36 | chr3:50312913 | |
| | | | | |
| cg13470920 | VAV1 | NM_005428 | chr19:6772831 | |

The correlation between the optimized 3-gene signature and intratumoral lymphocyte infiltration (TIL) was assessed utilizing the PNC1 breast cancer cohort, for which pathological TIL data and Infinum®HumanMethylation450 signatures were available. Figure 5 shows that the optimized MeTIL signature significantly anti-correlates with intratumoral lymphocyte infiltration (TIL).

Subsequently a more performant 5-gene signature was developed, comprising the methylation status of the 5 genes displayed in Table 4.

**Table 4 Abbreviations: RASSF1: Homo sapiens Ras association (RaIGDS/AF-6) domain family member 1; SEMA3B: Homo sapiens sema domain, immunoglobulin domain, secreted, (semaphorin) 3B domain (Ig), short basic; KLHL6: Homo sapiens kelch-like family member 6; PTPRCAP: Homo sapiens protein tyrosine phosphatase, receptor type, C-associated protein; INA: Homo sapiens internexin neuronal intermediate filament protein, alpha.**

| **IlluminaID** | **Corresponding gene** | **Correspondin g RefSeq mRNA ID** | **Chromosomal position cytosine** | **Target sequence** |
|---|---|---|---|---|
| cg00777121 | RASSF1 | NM_007182;NM_170714 | chr3:50378191 | |
| cg08047457 | RASSF1 | NM_007182;NM_170714 | chr3:50378413 | |
| | | | | |
| cg12069309 | SEMA3B | NM_001005914;NM_004636 | chr3:50312913 | |
| cg20425130 | KLHL6 | NM_130446 | chr3:183273245 | |
| cg20792833 | PTPRCAP | NM_005608 | chr11:67205195 | |
| cg21554552 | RASSF1 | NM_007182;NM_170714 | chr3:50378425 | |
| cg23642747 | INA | NM_032727 | chr10:105036645 | |

The correlation between the optimized signature and intratumoral lymphocyte infiltration (TIL) was assessed utilizing the discovery and validation (respectively cohort 1 and 2) breast cancer cohorts, for which pathological TIL data and Infinum®HumanMethylation450 signatures were available.

The MeTIL markers were identified in the following steps: 1) To identify markers for distinction between TIL and tumor cells we filtered for probes (CpG) from genome-wide DNA methylation profiles (Infinium Methylation) that are highly differential methylated (delta beta value < -0.8) between T lymphocytes and breast epithelial cells. 2) Applying a random forest machine learning approach on DNA methylation profiles (Infinium Methylation) from 105 breast primary tumors for which H&E staining-based pathological TIL readings were available to select the best signature from our list T cell specific CpGs that predicts most accurately the quantity of TIL in patient samples. The final signature was named 'MeTIL' (Methylation of TIL) signature.

Figure 6 shows that the individual genes selected from the group comprising: INA, PTPRCAP, SEMA3B, KLHL6 and RASSF1 MeT profiles outperform their respective expression profiles in terms of prediction of intratumoral lymphocyte infiltration (TIL). The markers of the MeTIL signature show highly differential methylation patterns in epithelial breast cells (blue) versus lymphocytes (orange). On the gene expression level inverse patterns appear that are less distinctive between epithelial breast cells (blue) and lymphocytes (orange) than methylation patterns.

When all five MeT profiles are combined in a single signature, the predictive power is even more increased as can be seen in Figure 7. In figure 7, the MeTIL score comprising the combined MeT profiles of all said genes (INA, PTPRCAP, SEMA3B, KLHL6 and RASSF1), is tested on different breast cancer types and compared to the known Pathological-TIL profile of said different cancer types, indicating that said MeTIL score outperforms the Patho-TIL score in terms of sensitivity. The MeTIL signature was transformed into a score and correlated with pathological TIL readings in cohort 1 (discovery cohort; 105 samples) and cohort 2 (independent validation cohort; 115 samples) breast cancer cohorts. The MeTIL score values are plotted on the y-axis and pathological TIL readings in % on the x-axis. The Spearman's rank correlation coefficient and its p value are displayed in the lower, right corner of each plot (Figure 7A).

Next, the levels of TIL in breast cancer subtypes were predicted based on the MeTIL score (left panel) and pathological TIL readings (right panel) in cohort 1 (discovery), cohort 2 and TCGA (The Cancer Genome Atlas cohort) breast cancer cohorts. Note, the MeTIL score shows greater differences in TIL levels within (especially in LumA and LumB) and between subtypes than pathological TIL readings suggesting a greater sensitivity of the MeTIL signature for detection of TIL (Figure 7B).

Subsequently, ROC curves were plotted to compare the specificity and sensitivity of prediction of survival and response to anthracycline treatment between the MeTIL and PathoTIL scores in different breast cancer types. Figure 8 shows the results: A) ROC curves for survival are shown for breast cancer subtypes (triple negative (TN), luminal (LUM), HER2-positive (HER2) based on the MeTIL score (orange) or pathological TIL readings (black) in various breast cancer cohorts (cohort 1, cohort 2, TCGA, TOP). B) ROC curves for survival in the HER2-enriched breast cancer subtype (defined with PAM50 signature) based on the MeTIL score (orange) or pathological TIL readings (black) in cohort 1 and TCGA cohort.

Figure 9 shows Forest plots by breast cancer subtype showing the log2 value of the hazard ratios (HR) and confidence intervals (CI) for prediction of survival outcome for the MeTIL score (orange) or pathological TIL readings (gray) in various breast cancer cohorts. Asterisks indicate statistical significance (p < 0.05).

Figure 10A shows the ROC curve for prediction of response to neoadjuvant anthracycline treatment based on the MeTIL score in 58 patients of the TOP cohort, compares the MeTIL and PathoTIL scores for predicting the response to anthracycline based chemotherapy. Figure 10B shows forest plot of the log2 value of the odds ratios (OR) and CI of the MeTIL score (orange) and various other clinical and pathological relevant variables (gray) for prediction of response to neoadjuvant anthracycline treatment in the TOP cohort. Asterisk indicates statistical significance (p < 0.05).

Next, we determined the MeTIL score in FFPE tumor tissue by bisulfite pyrosequencing. The use of pyrosequencing on very low amounts of DNA obtained from FFPE tumor tissue was compared to the use of the Infinium methylation microarray technology, showing that pyrosequencing is a cost-efficient alternative for Infinium to analyse the MeTIL score in patients. Figure 11 shows a scatter plot displaying the correlation between the MeTIL score determined by pyrosequencing in FFPE tumor tissue (y-axis) and the MeTIL score determined by Infinium arrays in fresh-frozen tumor tissue (x-axis). The correlation was established based on 21 patients of cohort 1, for which fresh-frozen and FFPE tissue was available. The Spearman's rank correlation coefficient (rho) and its p value are displayed in the lower, right corner of the plot.

The predictive value of the MeTIL signature for response to anthracycline treatment in comparison to gene expression-based immune markers was compared an displayed in Figure 12. **A)** ROC curves for prediction of response to neoadjuvant anthracycline treatment based on the MeTIL score or gene expression-based immune markers in 54 patients of the TOP cohort. **B)** Forest plot showing the log2 value of the odds ratios (OR) and CI of the MeTIL score (top row) or gene expression-based immune markers (others) for prediction of response to neoadjuvant anthracycline treatment in the TOP cohort. Asterisk indicates statistical significance (p < 0.05).

The following tables list the performance of the 5-gene MeTIL score compared to other known scores:

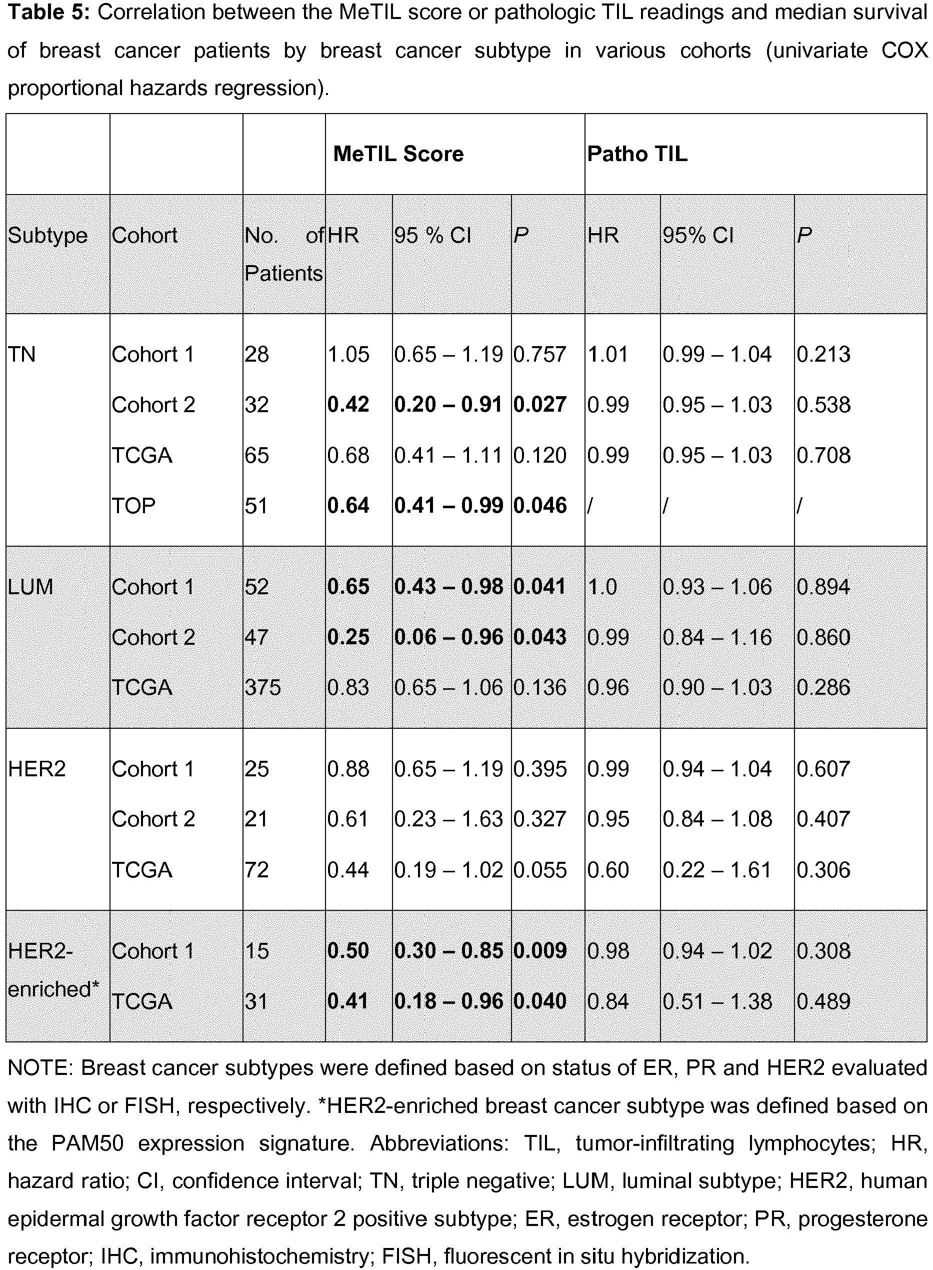

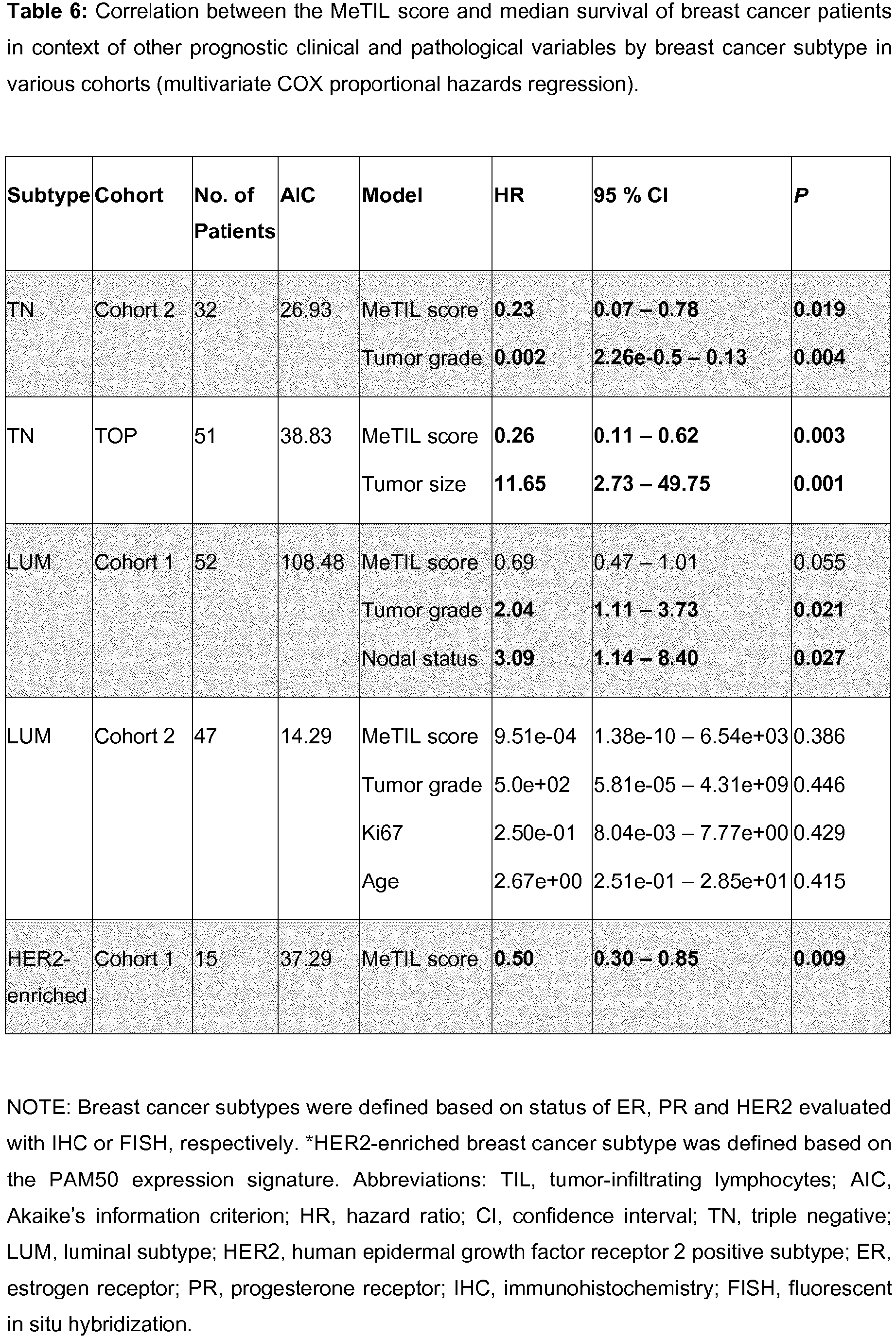

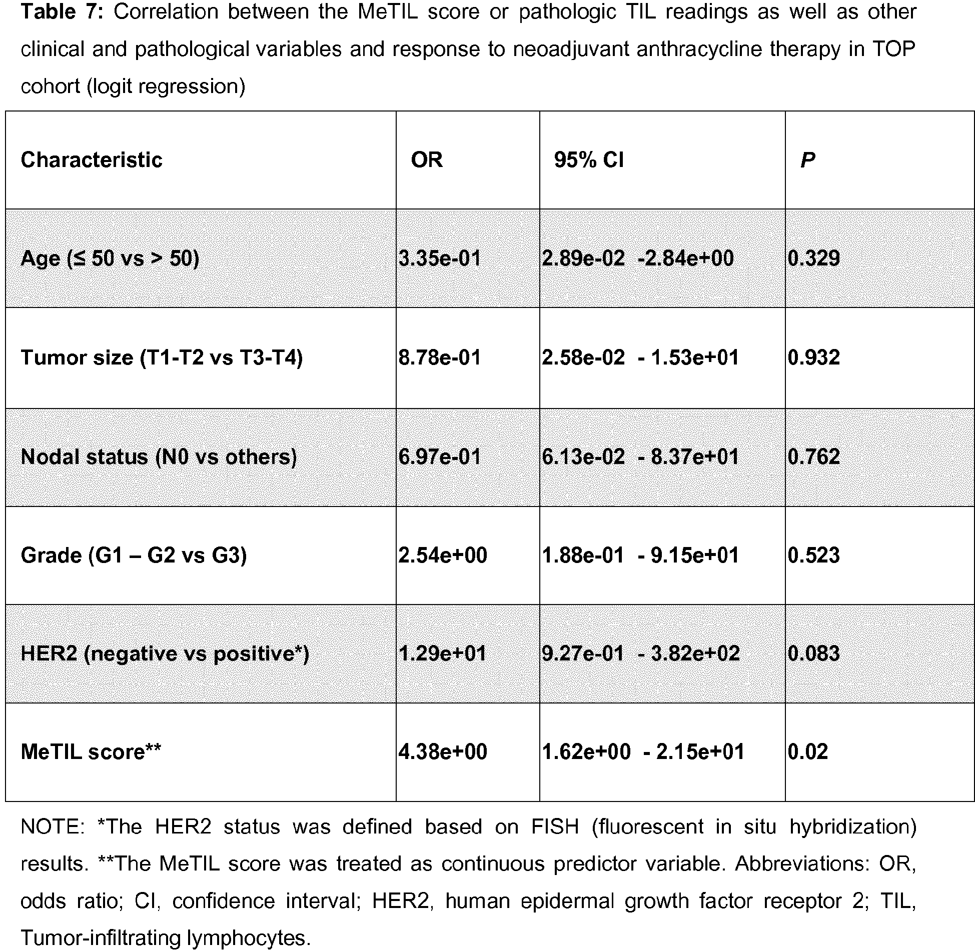

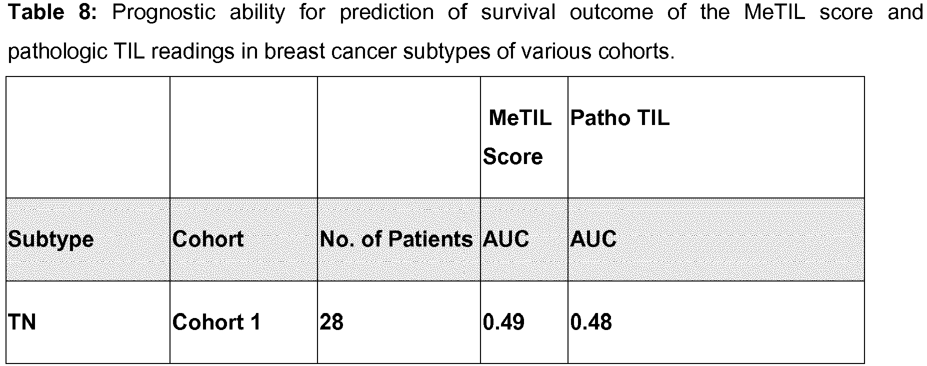

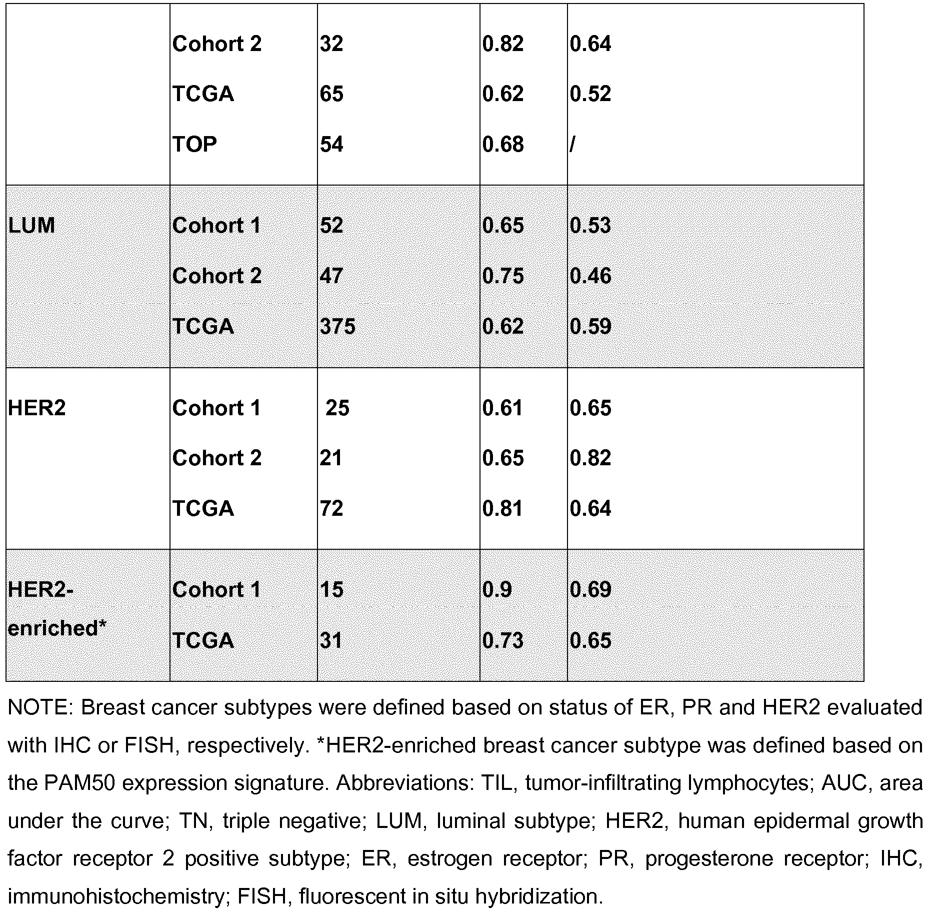

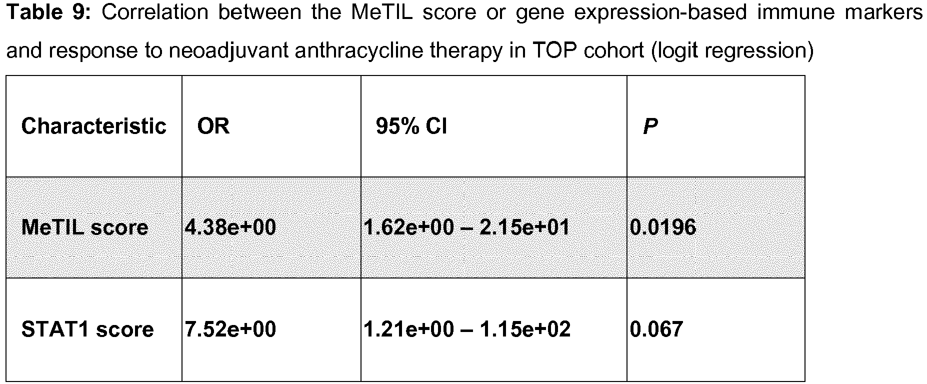

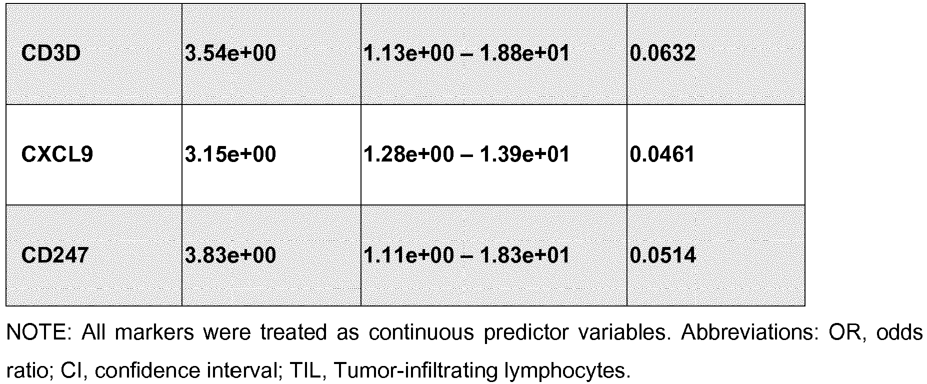

### References list

[1] Nabholtz JM, Riva A: Taxane/anthracycline combinations: setting a new standard in breast cancer? Oncologist 2001, 6(Suppl 3):5-12.
[2] Martin M, Pienkowski T, Mackey J, Pawlicki M, Guastalla JP, Weaver C, Tomiak E, Al-Tweigeri T, Chap L, Juhos E, Guevin R, Howell A, Fornander T, Hainsworth J, Coleman R, Vinholes J, Modiano M, Pinter T, Tang SC, Colwell B, Prady C, Provencher L, Walde D, Rodriguez-Lescure A, Hugh J, Loret C, Rupin M, Blitz S, Jacobs P, Murawsky M, Riva A, Vogel C, Breast Cancer International Research Group 001 Investigators: Adjuvant docetaxel for node-positive breast cancer. N Engl J Med 2005, 352:2302-2313.
[3] Levine MN, Pritchard KI, Bramwell VH, Shepherd LE, Tu D, Paul N. A randomized trial comparing cyclophosphamide, epirubicin, and fluorouracil with cyclophosphamide, methotrexate, and fluorouracil in premenopausal women with node-positive breast cancer: update of National Cancer Institute of Canada Clinical Trials Group MA5. J Clin Oncol 2005;23:5166-70.
[4] Early Breast Cancer Trialists' Collaborative Group (EBCTCG). Effects of chemotherapy and hormonal therapy for early breast cancer on recurrence and 15-year survival: an overview of the randomised trials. Lancet 2005;365:1687-717.
[5] Jones RL, Swanton C, Ewer MS: Anthracycline cardiotoxicity. Expert Opin Drug Saf 2006, 5:791-809.
[6] Cardoso F, Atalay G, Piccart MJ. Optimizing anthracycline therapy for node positive breast cancer. Am J Cancer 2002;1:257-68.
[7] Piccart-Gebhart MJ. Moving away from the "one shoe fits all" strategy: the key to future progress in chemotherapy. J Clin Oncol 2005;23:1611-3.
[8] Yen, P. S., et al. Cancer Res, 61:8375-8380, 2001.
[9] Yang, X., et a1. Endocr Relat Cancer, 8:115-127, 2001.
[10] Widschwendter & Jones, Oncogene, 21:5462-5482, 2002.
[11] Estelleret al. 2001
[12] Methylation and expression of human telomerase reverse transcriptase in ovarian and cervical cancer, 2004, Gynecologic Oncology, Vol 93, pp 407-416.
[13] The MAGE-A1 gene expression is not determined solely by methylation status of the promoter region in hematological malignancies, 2002, Leukemia Research, Vol 26, pp 1113-1118.
[14] Dietrich etal. BMC Cancer 2010, 10:247
[15] Zhonghua Zhong Liu Za Zhi. 2009 Apr;31(4):282-6.
[16] Clin Cancer Res 2009; 15:315-323.
[17] Int. J. Cancer: 124, 1727-1735 (2009)
[18] Castro-Rivera et al. PNAS, 11432-11437 (2014)
[19] Fackler et al. Cancer Research, 71(19):6195-6207 (2011)

## Claims

1. A method for predicting an anthracycline treatment outcome in a patient with breast cancer; said method comprising:
a) establishing a reference methylation signature comprises determining the methylation status of the INA RASSF1, SEMA3B, KLHL6, and PTPRCAP genes in a test sample from a responder or non-responder to anthracycline treatment, thereby producing a reference methylation signature of a responder or non-responder to anthracycline treatment;
b) assaying in a test sample from the patient the methylation status of the SEMA3B, PTPRCAP, INA, KLHL6, and RASSF1 gene;
c) calculating a methylation signature of the test sample based on the methylation status obtained in step b); and
d) comparing said test sample methylation signature with the reference methylation signature of a responder or non-responder obtained in step a), thereby preidicting the anthracycline treatment outcome in said patient.

2. The method according to claim 1, wherein said outcome results in predicting susceptibility to an anthracycline treatment in a patient with breast cancer.

3. The method according to claim 1, wherein said good outcome indicates that said patient will be responsive to an anthracycline treatment.

4. The method according to any one of claims 1 to 3, wherein the methylation status of the regions of said genes as defined by SEQ ID No 8, and optionally any one of SEQ ID Nos 1, 23, 11, 18, 20, or 21 is determined.

5. The method according to any one of claims 1 to 4, wherein the methylation status of at least one gene selected from the group comprising: SHANK2, FAM113B, VSX1, DRD4, CD6, BIN2, MT3, HEM1, LCK, EDG6, ADA, Cl6orf54, LAT, SH2D3C, CD3D, PPP1R16B, OSM, VAV1, and ALDH1A3 is further assayed in said test sample.

6. The method according to claim 5, wherein the methylation status of one or more regions of said genes as defined by SEQ ID Nos 1-29 is determined.

7. The method according to any of claims 1-6, wherein the methylation status of said genes or said region thereof, is determined by one or more techniques selected from the group comprising: nucleic acid amplification, polymerase chain reaction (PCR), methylation specific PCR (MCP), methylated-CpG island recovery assay (MIRA), combined bisulfite-restriction analysis (COBRA), bisulfite pyrosequencing, single-strand conformation polymorphism (SSCP) analysis, restriction analysis, microarray analysis, or bead-chip technology.

8. The method according to any of claims 1-7, wherein said anthracycline treatment encompasses anthracycline-based chemotherapy combinations.

9. Use of the methylation status of the SEMA3B, PTPRCAP, INA, KLHL6, and RASSF1 genes, as a marker or preclinical marker for anthracycline treatment response in a breast cancer patient and for assessing intratumoral lymphocyte infiltration in a breast cancer patient.

10. Anthracycline compound for use in the prevention or treatment of a breast cancer patient, **characterised in that** said patient has been found responsive by the method according to any one of claims 3 to 8.

11. Use of a set of oligonucleotides consisting of isolated oligonucleotides comprising a nucleotide sequence complementary to SEQ ID No 8, 1, 23, 11, 18, 20, and 21, for determining an anthracycline treatment outcome in a patient with breast cancer; for predicting susceptibility to an anthracycline treatment in a patient with breast cancer; or for the stratification of breast cancer patients.

## Patentansprüche

1. Verfahren zur Vorhersage des Ergebnisses einer Anthracyclinbehandlung bei einer Patientin mit Brustkrebs; wobei das Verfahren Folgendes umfasst:
a) Erstellen einer Referenz-Methylierungssignatur, umfasst Bestimmen des Methylierungsstatus der Gene INA RASSF1, SEMA3B, KLHL6 und PTPRCAP in einer Testprobe von einem auf Anthracyclinbehandlung ansprechenden oder nicht ansprechenden Individuum, womit eine Referenz-Methylierungssignatur eines auf Anthracyclinbehandlung ansprechenden oder nicht ansprechenden Individuums erzeugt wird;
b) Testen des Methylierungsstatus des SEMA3B-, PTPRCAP-, INA-, KLHL6- and RASSF1-Gens in einer Testprobe von der Patientin;
c) Berechnen einer Methylierungssignatur der Testprobe anhand des in Schritt b) erhaltenen Methylierungsstatus; und
d) Vergleichen der Testproben-Methylierungssignatur mit der in Schritt a) erhaltenen Referenz-Methylierungssignatur eines ansprechenden oder nicht ansprechenden Individuums, womit das Ergebnis der Anthracyclinbehandlung bei der Patientin vorhergesagt wird.

2. Verfahren nach Anspruch 1, wobei das Ergebnis zu einer Vorhersage von Suszeptibilität für eine Anthracyclinbehandlung bei einer Patientin mit Brustkrebs führt.

3. Verfahren nach Anspruch 1, wobei das gute Ergebnis anzeigt, dass die Patienten auf eine Anthracyclinbehandlung ansprechen wird.

4. Verfahren nach einem der Ansprüche 1 bis 3, wobei der Methylierungsstatus der Regionen der Gene gemäß SEQ ID Nr. 8 und gegebenenfalls eine von SEQ ID Nr. 1, 23, 11, 18, 20 oder 21 bestimmt wird.

5. Verfahren nach einem der Ansprüche 1 bis 4, wobei der Methylierungsstatus wenigstens eines Gens ausgewählt aus der Gruppe umfassend: SHANK2, FAM113B, VSX1, DRD4, CD6, BIN2, MT3, HEM1, LCK, EDG6, ADA, Cl6orf54, LAT, SH2D3C, CD3D, PPP1R16B, OSM, VAV1 und ALDH1A3 weiter in der Testprobe getestet wird.

6. Verfahren nach Anspruch 5, wobei der Methylierungsstatus einer oder mehrerer Regionen der Gene gemäß SEQ ID Nr. 1-29 bestimmt wird.

7. Verfahren nach einem der Ansprüche 1-6, wobei der Methylierungsstatus der Gene oder der Region davon mit einer oder mehreren Techniken bestimmt wird, die ausgewählt sind aus der Gruppe umfassend: Nukleinsäureamplifikation, Polymerasekettenreaktion (PCR), methylierungsspezifische PCR (MCP), MIRA (Methylated-CpG Island Recovery Assay), COBRA (Combined Bisulfite-Restriction Analysis), Bisulfit-Pyrosequenzierung, SSCP (Single-Strand Conformation Polymorphism)-Analyse, Restriktionsanalyse, Mikroarray-Analyse oder Bead-Chip-Technologie.

8. Verfahren nach einem der Ansprüche 1-7, wobei die Anthracyclinbehandlung Chemotherapiekombinationen auf Anthracyclinbasis umfasst.

9. Verwendung des Methylierungsstatus der Gene SEMA3B, PTPRCAP, INA, KLHL6 and RASSF1 als Marker oder vorklinischen Marker für Ansprechen auf Anthracyclinbehandlung bei einer Brustkrebspatientin und zur Beurteilung intratumoraler Lymphozyteninfiltration bei einer Brustkrebspatientin.

10. Anthracyclinverbindung zur Verwendung bei der Vorbeugung oder Behandlung einer Brustkrebspatientin, **dadurch gekennzeichnet, dass** mit dem Verfahren nach einem der Ansprüche 3 bis 8 ein Ansprechen der Patientin festgestellt wurde.

11. Verwendung eines Satzes von Oligonukleotiden, bestehend aus isolierten Oligonukleotiden, die eine zu SEQ ID Nr. 8, 1, 23, 11, 18, 20 und 21 komplementäre Nukleotidsequenz umfassen, zur Bestimmung des Ergebnisses einer Anthracyclinbehandlung bei einer Patientin mit Brustkrebs; zur Vorhersage von Suszeptibilität für eine Anthracyclinbehandlung bei einer Patientin mit Brustkrebs; oder für die Stratifizierung von Brustkrebspatientinnen.

## Revendications

1. Méthode de prédiction du résultat d'un traitement par l'anthracycline chez un patient souffrant d'un cancer du sein, ladite méthode comprenant :
a) l'établissement d'une signature de méthylation de référence comprenant la détermination de l'état de méthylation des gènes INA, RASSF1, SEMA3B, KLHL6 et PTPRCAP dans un échantillon à tester issu d'un répondeur ou non répondeur vis-à-vis d'un traitement par l'anthracycline, produisant ainsi une signature de méthylation de référence d'un répondeur ou non répondeur vis-à-vis d'un traitement par l'anthracycline ;
b) le dosage, dans un échantillon à tester issu du patient, de l'état de méthylation des gènes SEMA3B, PTPRCAP, INA, KLHL6 et RASSF1 ;
c) le calcul d'une signature de méthylation de l'échantillon à tester, sur la base de l'état de méthylation obtenu dans l'étape b) ; et
d) la comparaison de ladite signature de méthylation de l'échantillon à tester avec la signature de méthylation de référence d'un répondeur ou non répondeur obtenue dans l'étape a), prédisant ainsi le résultat d'un traitement par l'anthracycline chez ledit patient.

2. Méthode selon la revendication 1, dans laquelle ledit résultat conduit à la prédiction de la sensibilité vis-à-vis d'un traitement par l'anthracycline chez un patient souffrant d'un cancer du sein.

3. Méthode selon la revendication 1, dans laquelle ledit bon résultat indique que ledit patient sera répondeur vis-à-vis d'un traitement par l'anthracycline.

4. Méthode selon l'une quelconque des revendications 1 à 3, dans laquelle on détermine l'état de méthylation des régions desdits gènes tels que définis par SEQ ID n° 8, et éventuellement l'une quelconque parmi SEQ ID n° 1, 23, 11, 18, 20 ou 21.

5. Méthode selon l'une quelconque des revendications 1 à 4, dans laquelle l'état de méthylation d'au moins un gène choisi dans le groupe constitué par SHANK2, FAM113B, VSX1, DRD4, CD6, BIN2, MT3, HEM1, LCK, EDG6, ADA, Cl6orf54, LAT, SH2D3C, CD3D, PPP1R16B, OSM, VAV1 et ALDH1A3, est en outre dosé dans ledit échantillon à tester.

6. Méthode selon la revendication 5, dans laquelle on détermine l'état de méthylation d'une ou plusieurs régions desdits gènes tels que définis par SEQ ID n° 1-29.

7. Méthode selon l'une quelconque des revendications 1-6, dans laquelle l'état de méthylation desdits gènes ou de ladite région de ceux-ci, est déterminé par une ou plusieurs techniques choisies dans le groupe constitué par l'amplification d'acide nucléique, l'amplification en chaîne par polymérase (PCR), la PCR spécifique de la méthylation (MCP), le dosage de récupération d'ilot CpG méthylé (MIRA), l'analyse par restriction et bisulfite combinés (COBRA), le pyroséquençage au bisulfite, l'analyse du polymorphisme de conformation des simples brins (SSCP), l'analyse par restriction, l'analyse par puce à ADN, ou la technologie BeadChip.

8. Méthode selon l'une quelconque des revendications 1-7, dans laquelle ledit traitement par l'anthracycline englobe des combinaisons de chimiothérapie à base d'anthracycline.

9. Utilisation de l'état de méthylation des gènes SEMA3B, PTPRCAP, INA, KLHL6 et RASSF1, comme marqueur ou marqueur préclinique d'une réponse à un traitement par l'anthracycline chez un patient souffrant d'un cancer du sein, et pour évaluer l'infiltration lymphocytaire intra-tumorale chez un patient souffrant d'un cancer du sein.

10. Composé d'anthracycline pour une utilisation dans la prévention ou le traitement d'un patient souffrant d'un cancer du sein, **caractérisé en ce qu'**on a trouvé que ledit patient est répondeur par la méthode selon l'une quelconque des revendications 3 à 8.

11. Utilisation d'un set d'oligonucléotides constitué d'oligonucléotides isolés comprenant une séquence nucléotidique complémentaire de SEQ ID n° 8, 1, 23, 11, 18, 20 et 21, pour la détermination du résultat d'un traitement par l'anthracycline chez un patient souffrant d'un cancer du sein ; pour prédire la sensibilité vis-à-vis d'un traitement par l'anthracycline chez un patient souffrant d'un cancer du sein ; ou pour la stratification de patients souffrant d'un cancer du sein.


### SEQUENCE LISTING

<110> Université Libre de Bruxelles
<120> BREAST CANCER EPIGENETIC MARKERS USEFUL IN ANTHRACYCLINE TREATMENT PROGNOSIS
<130> ULB-066-PCT
<150> EP 14167401.0
   <151> 2014-05-07
<160> 29
<170> PatentIn version 3.5
<210> 1
   <211> 122
   <212> DNA
   <213> Homo Sapiens
<400> 1
   gcgcgatgcg cagcgcgttg gcacgctcca gccgggtgcg gcccttccca gcgcgcccag 60
   cgggtgccag ctcccgcagc tcaatgagct caggctcccc cgacatggcc cggttgggcc 120
   cg 122
<210> 2
   <211> 122
   <212> DNA
   <213> Homo Sapiens
<400> 2
   ggcaaagaaa gtaagtggcc cgaacggcgg cggcggcagc ggcggcctca gcagcggcgg 60
   cgtcgggaat gagctcctcc ggcttgtccc gaccacacgc atcttccagg gaagcccggg 120
   aa 122
<210> 3
   <211> 122
   <212> DNA
   <213> Homo Sapiens
<400> 3
   gacttaccat ttcatgtttt cacacatgtc aaagtcttga agcttcggtc attagtcttc 60
   cgcttcctaa agcaagaaac taaatgctga cttgtgtcgt ttgccccaga tgccaccgga 120
   aa 122
<210> 4
   <211> 122
   <212> DNA
   <213> Homo Sapiens
<400> 4
   ccgcctagat ctgggccgct ggggccaggg gtgcggtggg gcgatggtct gtgacccctg        60
   cgcggctcag agcctagggg acaggggcag gagcggaaag cgcgggcctg attaccggac       120
   gt                                                                      122
<210> 5
   <211> 122
   <212> DNA
   <213> Homo Sapiens
<400> 5
   cgtgcccgtc tctccctgcg caaaattcca agatgagcaa atactgggct cacggtggag        60
   cgccgcgggg gcccccctga gccggggcgg gtcgggggcg ggaccagggt ccggccgggg       120
   cg                                                                      122
<210> 6
   <211> 122
   <212> DNA
   <213> Homo Sapiens
<400> 6
   tgcagaccaa aaccacaagc agaacaagca ggcgtgagac actcacaggt tgggtttgat        60
   cgcatgcgtg tcggagagga gagagcagag agagacacag gaacaagaac agcaaagggt       120
   ag                                                                      122
<210> 7
   <211> 122
   <212> DNA
   <213> Homo Sapiens
<400> 7
   aatgggcgca gggcgcggtg tatgcccgct cctcctcctg ttttcttcga attcgttctt        60
   cgaggtcagc cctacgccca aggatgatgt aaaaccgcag cctcagcctt cctagggtga       120
   gt                                                                      122
<210> 8
   <211> 122
   <212> DNA
   <213> Homo Sapiens
<400> 8
   aaggagctga ggagagccgc gcaatggaaa cctgggtgca gggactgtgg ggcccgaagg 60
   cggggctggg cgcgctctcg cagagccccc cccgccttgc ccttccttcc ctccttcgtc 120
   cc 122
<210> 9
   <211> 122
   <212> DNA
   <213> Homo Sapiens
<400> 9
   ccaaaaccac aagcagaaca agcaggcgtg agacactcac aggttgggtt tgatcgcatg 60
   cgtgtcggag aggagagagc agagagagac acaggaacaa gaacagcaaa gggtagagca 120
   ga 122
<210> 10
   <211> 122
   <212> DNA
   <213> Homo Sapiens
<400> 10
   tctctccaat ctgccagttt tcatggaagc ccactcctcg ctcccgtttc cctgggagag 60
   cgggagaccc ttctcgtcag cccagaccct tcggggcccc tgagcaccct gcccgcccct 120
   cc 122
<210> 11
   <211> 122
   <212> DNA
   <213> Homo Sapiens
<400> 11
   ggggtcgcgt gcacgcgctt ccagcccagt gccaagaggt gggcggggtc ggggttgggc 60
   cgccgggagg gaggcgaagg gtctttcact gcccggggct gaaagaaggg ctcacagaag 120
   at 122
<210> 12
   <211> 122
   <212> DNA
   <213> Homo Sapiens
<400> 12
   tggtggaggc tgcgagggtg cacggccggc cctgggcagg cggtagccat ggagctgtgg 60
   cgccaatgca cccactggct catccagtgc cgggtgctgc cgcccagcca ccgcgtgacc 120
   tg 122
<210> 13
   <211> 122
   <212> DNA
   <213> Homo Sapiens
<400> 13
   accgctcacc accaccccca acggggcacc ggcagccacg tggtcggaag gggcctatgg 60
   cgcaatggcc cggaaggcag tgacaccatc gttcccgtcc taagaaccat ccggcccttt 120
   gg 122
<210> 14
   <211> 122
   <212> DNA
   <213> Homo Sapiens
<400> 14
   cagggaagag ctgggaaata cgcaaagcgc ctttttctcc actttcggag atggtacgtg 60
   cgcgcttcca cgcagtggcg gctgctgcgg cgagcacgtc ccctgcggga cccacgcggg 120
   ga 122
<210> 15
   <211> 122
   <212> DNA
   <213> Homo Sapiens
<400> 15
   atgtctttga catctgctta ccagcataaa ttagcagaga agctcactat cctgaatgat 60
   cgcggtcagg gggttctcat ccgtatgtat aacatcaaga aggtaagcat gaacaatggg 120
   ac 122
<210> 16
   <211> 122
   <212> DNA
   <213> Homo Sapiens
<400> 16
   aatggggcca gagggctccc gggctgggca ggtaaggagc gctggtattg ggggcgcagg 60
   cgccggggtg agaggcctga tagcagacgg ctgcagctgt gcgggcccag gctccctagg 120
   ga 122
<210> 17
   <211> 122
   <212> DNA
   <213> Homo Sapiens
<400> 17
   ggccgggcgc ggggggccgg aggatggcgg cctgggggcc ctgcgggggc tgtcggtggc 60
   cgccagctgc ctggtggtgc tggagaactt gctggtgctg gcggccatca ccagccacat 120
   gc 122
<210> 18
   <211> 122
   <212> DNA
   <213> Homo Sapiens
<400> 18
   tggcaggaga attcctgaat gtccacacac aagatgacat ctgtcagagc gttttccatt 60
   cgcagggttt ccaggccatt ctgaagaatt aaggagagtc ccgcgtcgtc aaatttgacc 120
   tt 122
<210> 19
   <211> 122
   <212> DNA
   <213> Homo Sapiens
<400> 19
   ttcttccccg gggcgcatcc acaaacctga gctccttggc gctcaccacc ataacgctgg 60
   cgtacaaagc tgcttccatt cactgagcac tcagtaggcg ccgggcacag cactaggtgc 120
   ct 122
<210> 20
   <211> 122
   <212> DNA
   <213> Homo Sapiens
<400> 20
   gctgtgtcga gcgagaagtg agctcagtgc tcgtctgcag tgaagggtgg cccaggcttc 60
   cgcttcctgc ccacataccc cacctgcccc tccctgctgc aggacccctg gtccacacca 120
   ga 122
<210> 21
   <211> 122
   <212> DNA
   <213> Homo Sapiens
<400> 21
   agagccgcgc aatggaaacc tgggtgcagg gactgtgggg cccgaaggcg gggctgggcg 60
   cgctctcgca gagccccccc cgccttgccc ttccttccct ccttcgtccc ctcctcacac 120
   cc 122
<210> 22
   <211> 122
   <212> DNA
   <213> Homo Sapiens
<400> 22
   tagagccact cttcctggtg ctggactaag aggtgcaggc ttggagggtg cagggcggtc 60
   cgcctctcag acgtagaggc ccggcctcgg atgaaggcgg aagggagggc accgcctgtt 120
   gc 122
<210> 23
   <211> 122
   <212> DNA
   <213> Homo Sapiens
<400> 23
   gggatgcgac agagctgtgt ggtttccgga tgggaaacct cagtcgttta ggcacccctc 60
   cgctcgagtc acttccgaag cagtcgattc ttggggagaa gcgctgcgga aaggggcgac 120
   tc 122
<210> 24
   <211> 122
   <212> DNA
   <213> Homo Sapiens
<400> 24
   gcgactgaga tgggggactg ctcttctccg agtgagctgg ctgagtgtga ccgtgagtca 60
   cgcccctgct tccctggagc ctgtcccttg cctcacaggc ctggctgagg tgggggtggg 120
   ca 122
<210> 25
   <211> 122
   <212> DNA
   <213> Homo Sapiens
<400> 25
   ctggtcttct tggtcccctc tgtcatcttg gcaaattgtg tgaagccctt ggccagcttg 60
   cgagtggcca ctcaaggctc gggacatcaa agttgaggtg cctctcccac ctccccttcc 120
   cc 122
<210> 26
   <211> 122
   <212> DNA
   <213> Homo Sapiens
<400> 26
   tgcgagagaa gggtagccag taccaggcca gagagaaacg tgctatgttc catctcccag        60
   cggaactcat ccagtagata aagccaggtc accgaactat cagcctgggt gagagctgcc       120
   ct                                                                      122
<210> 27
   <211> 122
   <212> DNA
   <213> Homo Sapiens
<400> 27
   ttcctggtgc tggactaaga ggtgcaggct tggagggtgc agggcggtcc gcctctcaga        60
   cgtagaggcc cggcctcgga tgaaggcgga agggagggca ccgcctgttg ctgggcaact       120
   gt                                                                      122
<210> 28
   <211> 122
   <212> DNA
   <213> Homo Sapiens
<400> 28
   tacgaccact tccttcctcc agcgcttcac ctcccttctt tccagaaaga ggaaatgatg        60
   cgagtccaca ccgcaggcca cagcccccat gtctcggccc cagcagctgg gggacctggc       120
   cc                                                                      122
<210> 29
   <211> 122
   <212> DNA
   <213> Homo Sapiens
<400> 29
   gcctcctcgc tctgtgggct gggaggctac agcgttggca gaacggcgag attacctgcc        60
   cggggcagaa aggaataaaa gaagatggtg aggcctccga ggcctctgag ctggggctga       120
   gg                                                                      122